# EUROPEAN PATENT APPLICATION

(11) **EP 4 524 547 A1**
(43) Date of publication of application: **19.03.2025**
(21) Application number: 24199748.5
(22) Date of filing: 11.09.2024
(51) Int. Cl.: G01N 15/01, G01N 15/10, G01N 15/14, G01N 15/1429, G01N 21/64, G01N 33/58

(54) **SPECIMEN ANALYZER AND SPECIMEN ANALYSIS METHOD**

(30) Priority: 13.09.2023 JP 2023148647
(71) Applicant: SYSMEX CORPORATION, Kobe-shi, Hyogo 651-0073 (JP); University of Toyama, Toyama 930-8555 (JP)
(72) Inventor: YOSHIDA, Rinako, Kobe-shi, Hyogo, 651-0073 (JP); IWASAKI, Yosuke, Kobe-shi, Hyogo, 651-0073 (JP); NIIMI, Hideki, Toyama-shi, Toyama, 930-0194 (JP); YAMAMOTO, Yoshihiro, Toyama-shi, Toyama, 930-0194 (JP); MIYAJIMA, Yuki, Toyama-shi, Toyama, 930-0194 (JP); KANEDA, Makito, Toyama-shi, Toyama, 930-0194 (JP)
(74) Representative: Hoffmann Eitle

(57) **Abstract**

Disclosed is a specimen analyzer comprising: a measurement part configured to apply light to a measurement sample containing a blood specimen and a fluorescent dye that stains white blood cells contained in the blood specimen, detect fluorescence from the white blood cells contained in the measurement sample to which the light has been applied, and measure fluorescence signals on the basis of the detected fluorescence; an information processing part configured to generate, on the basis of the fluorescence signals, information about a distribution width of fluorescence intensities regarding a population of neutrophils contained in the measurement sample, and determine information about a degree of severity of an organ dysfunction due to an infection on the basis of the information about the distribution width of the fluorescence intensities regarding the population of neutrophils; and an output part configured to output the information about the degree of severity of the organ dysfunction due to the infection.

## Description

### CROSS REFERENCE TO RELATED APPLICATIONS

This application claims priority from prior Japanese Patent Application No. 2023-148647, filed on September 13, 2023, entitled "SPECIMEN ANALYSIS METHOD AND SPECIMEN ANALYZER", the entire content of which is incorporated herein by reference.

### FIELD OF THE INVENTION

The present invention relates to a specimen analyzer for analyzing a specimen and a specimen analysis method.

### BACKGROUND OF THE INVENTION

Sabrina Buoro, et al. "Clinical significance of cell population data (CPD) on Sysmex XN-9000 in septic patients with our without liver impairment", Annals of Translational Medicine, November 2016, Vol. 4, No. 21, p. 1-9 discloses that, among data of various types of white blood cells obtained by the automatic multi-item blood cell analyzer XN-9000 (manufactured by Sysmex Corporation), fluorescence intensities (NE-SFL) obtained from neutrophils each having an average amount of nucleic acid and scattered light intensities (MO-X) obtained from monocytes each having an average morphological feature are correlated with sequential organ failure assessment (SOFA) score indicating the extent of an organ dysfunction.

The method of the SOFA score is a method for evaluating a degree of severity of an organ dysfunction due to an infection, the method having been advocated by the European Society of Intensive Care Medicine (ESICM). The SOFA score is widely used in ICUs. Calculation of a SOFA score requires execution of a plurality of tests including tests for the respiratory system, coagulability, the liver, the circulatory system, the central nerves, and the renal function. Accordingly, it takes a long time to calculate the score. Against this drawback, Sabrina Buoro, et al. "Clinical significance of cell population data (CPD) on Sysmex XN-9000 in septic patients with our without liver impairment", Annals of Translational Medicine, November 2016, Vol. 4, No. 21, p. 1-9 proposes, as a swifter test method, a method in which the NE-SFL and the MO-X obtained by the automatic multi-item blood cell analyzer XN-9000 are used.

However, a Spearman's rank correlation coefficient between the SOFA score and each of the NE-SFL and the MO-X described in Sabrina Buoro, et al. "Clinical significance of cell population data (CPD) on Sysmex XN-9000 in septic patients with our without liver impairment", Annals of Translational Medicine, November 2016, Vol. 4, No. 21, p. 1-9, is 0.4 or smaller. Thus, the method in which the NE-SFL and the MO-X are used has not yet been employed in clinical sites as a method for evaluating a degree of severity of an organ dysfunction due to an infection.

In view of this circumstance, it has been required to provide a specimen analysis method and a specimen analyzer for performing assistance in a swift evaluation of a degree of severity of an organ dysfunction due to an infection, the specimen analysis method and the specimen analyzer enabling outputting of information having a high correlation with a score indicating the degree of severity of the organ dysfunction due to the infection.

### SUMMARY OF THE INVENTION

A specimen analyzer (1) of the present invention includes: a measurement part (2) configured to apply light to a measurement sample containing a blood specimen and a fluorescent dye that stains white blood cells contained in the blood specimen, detect fluorescence from the white blood cells contained in the measurement sample to which the light has been applied, and measure fluorescence signals on the basis of the detected fluorescence; an information processing part (40) configured to generate, on the basis of the measured fluorescence signals, information about a distribution width of fluorescence intensities regarding a population of neutrophils contained in the measurement sample and determine information about a degree of severity of an organ dysfunction due to an infection on the basis of the information about the distribution width of the fluorescence intensities regarding the population of neutrophils; and an output part (42) configured to output the information about the degree of severity of the organ dysfunction due to the infection.

A specimen analysis method of the present invention includes: applying light to a measurement sample containing a blood specimen and a fluorescent dye that stains white blood cells contained in the blood specimen, detecting fluorescence from the white blood cells contained in the measurement sample to which the light has been applied, and measuring fluorescence signals on the basis of the detected fluorescence; generating, on the basis of the fluorescence signals, information about a distribution width of fluorescence intensities regarding a population of neutrophils contained in the measurement sample; determining information about a degree of severity of an organ dysfunction due to an infection on the basis of the information about the distribution width of the fluorescence intensities regarding the population of neutrophils; and outputting the information about the degree of severity of the organ dysfunction due to the infection.

The present invention makes it possible to provide a specimen analysis method and a specimen analyzer for performing assistance in a swift evaluation of a degree of severity of an organ dysfunction due to an infection, the specimen analysis method and the specimen analyzer enabling outputting of information having a high correlation with a score indicating the degree of severity of the organ dysfunction due to the infection.

### BRIEF DESCRIPTION OF THE DRAWINGS

FIG. 1 is a perspective view showing the appearance of a blood cell analyzer according to a first embodiment of the present invention;
FIG. 2 shows a configuration of a measurement unit;
FIG. 3 shows a configuration of an optical detector;
FIG. 4 shows a configuration of an information processing unit;
FIG. 5A shows an example of a scattergram;
FIG. 5B shows an example of the scattergram having been subjected to demarcation processing;
FIG. 6 shows graphs of information (NE-WY) about distribution widths of fluorescence intensities regarding populations of neutrophils of hospitalized patients who suffered from sepsis (sepsis patients) and hospitalized patients who did not suffer from sepsis (non-sepsis patients) with respect to the number of days having elapsed after hospitalization, and a graph of information (NE-WY) about distribution widths of fluorescence intensities regarding populations of neutrophils of healthy individuals;
FIG. 7 shows a correlation between the information (NE-WY) about the distribution widths of the fluorescence intensities regarding the populations of neutrophils of the hospitalized patients (the sepsis patients and the non-sepsis patients) on the zeroth day after hospitalization and the SOFA score on said day;
FIG. 8 shows correlations each of which is a correlation between information (NE-WY) about the distribution widths of the fluorescence intensities regarding the populations of neutrophils on a corresponding one of days and the SOFA score on said day;
FIG. 9 shows an example of a first conversion table in which the information (NE-WY) about the distribution width of the fluorescence intensities regarding the population of neutrophils and estimated SOFA score are associated with each other;
FIG. 10A shows an example of an analysis result screen regarding a blood specimen according to the first embodiment of the present invention;
FIG. 10B shows another example of the analysis result screen regarding the blood specimen according to the first embodiment of the present invention;
FIG. 11 shows an example of a flowchart of operation by the information processing unit;
FIG. 12 shows an example of an analysis result screen regarding the blood specimen according to a second embodiment of the present invention;
FIG. 13 shows an example of a second conversion table in which the information (NE-WY) about the distribution widths of the fluorescence intensities regarding the population of neutrophils and the possibility of sepsis are associated with each other in a plurality of levels;
FIG. 14 shows an example of an analysis result screen regarding the blood specimen according to a third embodiment of the present invention;
FIG. 15 shows an example of an analysis result screen regarding the blood specimen according to a fourth embodiment of the present invention; and
FIG. 16 shows correlations each of which is a correlation between the information (NE-WY) about the distribution widths of the fluorescence intensities regarding the populations of neutrophils of the hospitalized patients on the corresponding day and the difference between the SOFA score on said day and the SOFA score in a healthy state.

### DETAILED DESCRIPTION

Specimen analyzers according to embodiments will be described with reference to the drawings. Each of the specimen analyzers according to the embodiments is a blood cell analyzer for analyzing blood cells in a blood specimen.

### [1. First Embodiment]

### [1-1. Schematic Configuration]

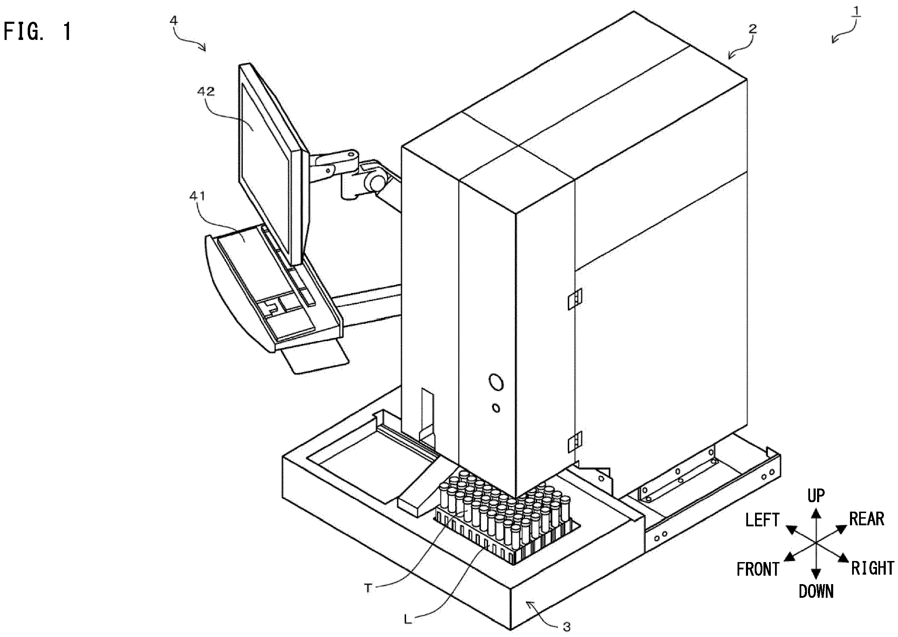

FIG. 1 is a perspective view showing the appearance of a blood cell analyzer according to a first embodiment. A blood cell analyzer 1 is a device for analyzing blood cells in a blood specimen. Specifically, the blood cell analyzer 1 is a multi-item blood cell analyzer for: classifying white blood cells contained in the blood specimen into different types; counting the numbers of the respective types of white blood cells; counting red blood cells and platelets; and measuring a hemoglobin concentration. The counting of red blood cells and platelets, and the measurement of a hemoglobin concentration, will not be described below. The counting of red blood cells and platelets, and the measurement of a hemoglobin concentration, can be performed by, for example, a measurement part described in US Patent No. 8,968,653. US Patent No. 8,968,653 is incorporated herein by reference.

The blood cell analyzer 1 includes a measurement unit 2, a transport unit 3, and an information processing unit 4.

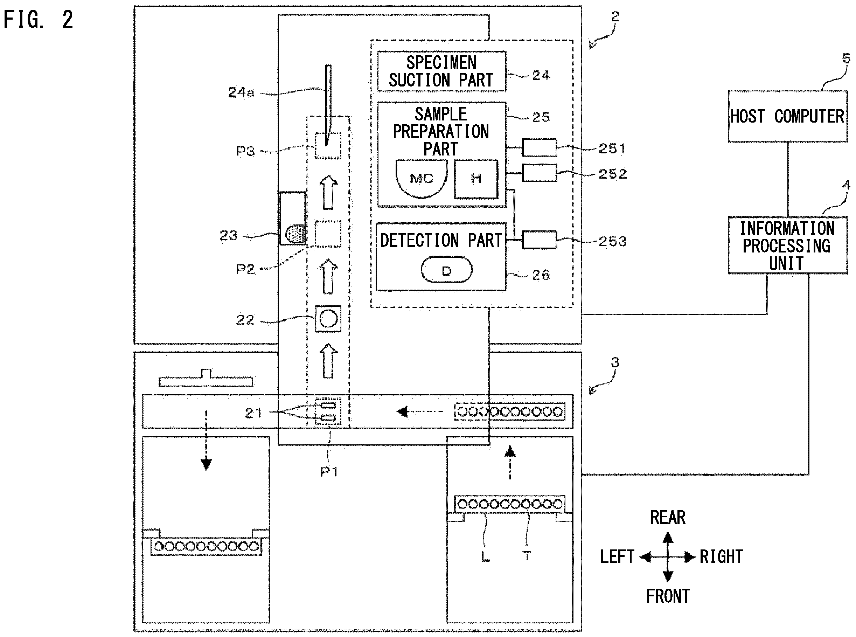

The transport unit 3 is disposed on the front surface of the measurement unit 2 and supplies a blood specimen to the measurement unit 2. Specifically, the transport unit 3 transports, to a position P1 (see FIG. 2) in front of the measurement unit 2, a sample rack L supporting a plurality of specimen containers (blood collection tubes) T each containing a blood specimen. The blood specimen is peripheral blood collected from a subject. The subject is one from whom peripheral blood is to be collected and is, for example, a patient. The patient is, for example, one having the possibility of developing an organ dysfunction due to an infection.

The measurement unit 2 mixes, with the blood specimen, a reagent containing a fluorescent dye for staining white blood cells, to prepare a measurement sample for use in measurement. The measurement unit 2 applies light to the prepared measurement sample and measures scattered light signals and fluorescence signals regarding the white blood cells contained in the measurement sample. The measurement unit 2 is communicably connected to the information processing unit 4 and transmits the result of the measurement to the information processing unit 4.

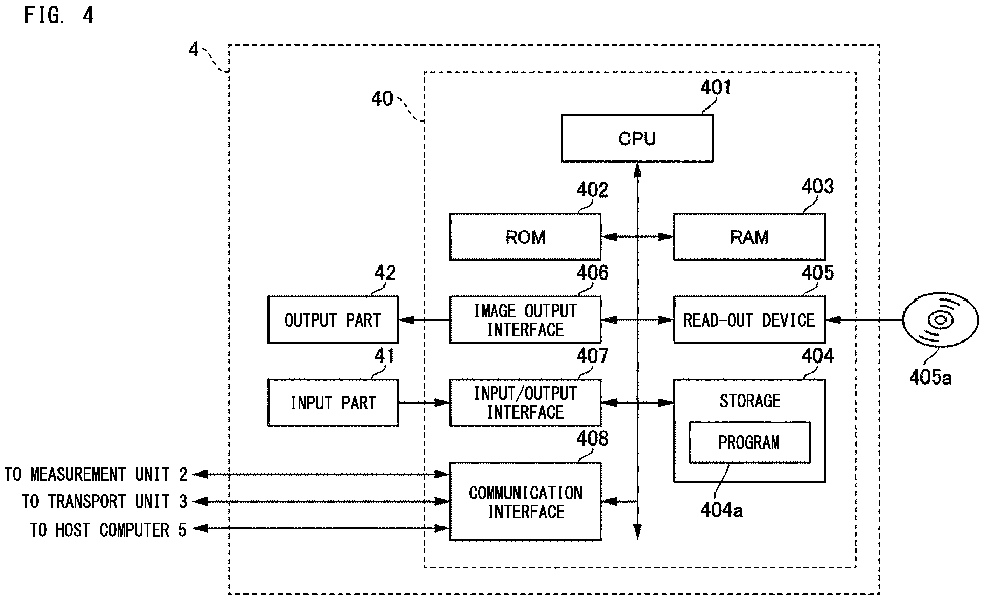

The information processing unit 4 is communicably connected to the measurement unit 2, the transport unit 3, and a host computer 5 (see FIG. 2). The information processing unit 4 controls operations to be performed by the measurement unit 2 and the transport unit 3, performs analysis on the basis of the result of the measurement performed by the measurement unit 2, causes an output part 42 to output the result of the analysis, and transmits the result of the analysis to the host computer 5. The information processing unit 4 includes a main body 40 (see FIG. 4), an input part 41, and the output part 42. The input part 41 is composed of, for example, a keyboard and a mouse. The output part 42 is, for example, a display. The display may be, for example, a liquid crystal display, an organic electroluminescence display, or a mini-LED display. The input part 41 and the output part 42 may be implemented by a touch panel.

### [1-2. Detailed Configuration]

### (Measurement Unit)

FIG. 2 shows a configuration of the measurement unit. The measurement unit 2 includes hand parts 21, a specimen container setting part 22, a barcode unit 23, a specimen suction part 24, a sample preparation part 25, and a detection part 26. These constituents 21 to 26 are controlled by the information processing unit 4.

The hand parts 21 grip any specimen container T positioned at the position P1, pulls out the specimen container T from the sample rack L, and swings the specimen container T. The specimen container setting part 22 receives, above the position P1, the specimen container T swung by the hand parts 21. The barcode unit 23 reads a specimen number from a barcode label pasted on the specimen container T. The specimen number is information to be associated with a result of analysis regarding the blood specimen. The specimen suction part 24 includes a suction tube 24a through which the blood specimen is suctioned from the specimen container T. The sample preparation part 25 includes a mixing chamber MC and a heater H and mixes a reagent with the blood specimen (hereinafter, sometimes referred to simply as "specimen") suctioned from the specimen container T, to prepare a measurement sample for use in measurement. The detection part 26 includes an optical detector D which detects fluorescence and scattered lights from white blood cells in the measurement sample.

These operations by the measurement unit 2 will be described, and the sample preparation part 25 and the detection part 26 will be described in detail.

The specimen container T positioned at the position P1 by the transport unit 3 is gripped and pulled out in the upward direction from the sample rack L by the hand parts 21. Then, the hand parts 21 are swung, to agitate the specimen in the specimen container T. The specimen container T having finished being agitated is set, by the hand parts 21, on the specimen container setting part 22 positioned above the position P1. Thereafter, the specimen container T is transported to a position P2 by the specimen container setting part 22.

When the specimen container T is positioned at the position P2, the barcode unit 23 disposed near the position P2 reads a specimen number from the barcode label pasted on the specimen container T. Thereafter, the specimen container T is transported to a position P3 by the specimen container setting part 22. When the specimen container T is positioned at the position P3, a predetermined amount of the specimen is suctioned from the specimen container T through the suction tube 24a by the specimen suction part 24. When the suctioning of the specimen is finished, the specimen container T is transported frontward by the specimen container setting part 22 and is returned to the original support position on the sample rack L by the hand parts 21. After the suction tube 24a is transferred to the position of the mixing chamber MC, a predetermined amount of the specimen suctioned through the suction tube 24a is ejected into the mixing chamber MC by the specimen suction part 24.

The sample preparation part 25 is connected via tubes to a reagent container 251 containing a first reagent, a reagent container 252 containing a second reagent, and a reagent container 253 containing a sheath liquid (diluent). In addition, the sample preparation part 25 is connected to a compressor (not shown) and can obtain a portion of each of the reagents from the corresponding one of the reagent containers 251 to 253 by means of a pressure generated by the compressor. The sample preparation part 25 mixes the blood specimen and each of the reagents with each other in the mixing chamber MC and heats the resultant liquid mixture for a predetermined time by the heater H, to prepare a measurement sample. The measurement sample prepared by the sample preparation part 25 is supplied to the optical detector D of the detection part 26.

The reagents include: the first reagent for staining the nucleic acids of white blood cells; and the second reagent for hemolyzing red blood cells and damaging the cell membranes of white blood cells to such an extent that a fluorescent dye can be transmitted through the cell membranes.

The first reagent is a reagent that contains a fluorescent dye capable of staining the nucleic acids and that is for fluorescently staining the nucleic acids of nucleated cells such as white blood cells in a blood sample treated with the second reagent described later. By treating a blood sample with the first reagent, blood cells having nucleic acids such as white blood cells are stained.

The fluorescent dye is not particularly limited as long as the fluorescent dye can stain such nucleic acids. The fluorescent dye can be selected as appropriate according to the wavelength of light to be applied from a light source (a semiconductor laser D31 described later). Examples of such a fluorescent dye include propidium iodide, ethidium bromide, ethidium-acridine heterodimer, ethidium diazide, ethidium homodimer-1, ethidium homodimer-2, ethidium monoazide, trimethylenebis[[3-[[4-[[(3-methylbenzothiazol-3-ium)-2-yl]methylene]-1,4-dihydroquinolin]-1-yl]propyl]dimethylaminium] tetraiodide (TOTO-1), 4-[(3-methylbenzothiazol-2(3H)-ylidene)methyl]-1-[3-(trimethylaminio)propyl]quinolinium diiodide (TO-PRO-1), N,N,N',N'-tetramethyl-N,N'-bis[3-[4-[3-[(3-methylbenzothiazol-3-ium)-2-yl]-2-propenylidene]-1,4-dihydroquinolin-1-yl]propyl]-1,3-propanediaminium tetraiodide (TOTO-3), 2-[3-[[1-[3-(trimethylaminio)propyl]-1,4-dihydroquinolin]-4-ylidene]-1-propenyl]-3-methylbenzothiazol-3-ium diiodide (TO-PRO-3), and fluorescent dyes represented by the following general formula (I). Among these fluorescent dyes, fluorescent dyes represented by the general formula (I) are preferable.

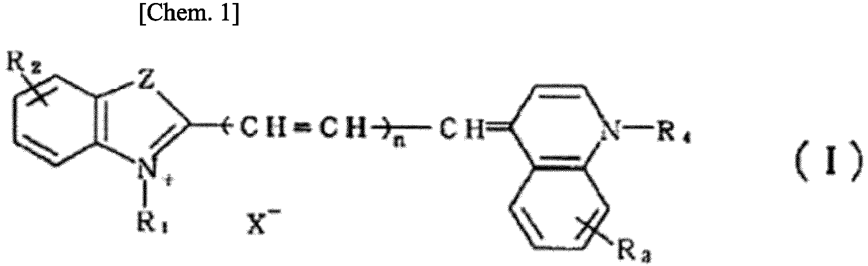

In the above formula (I), R₁ and R₄ are identical to or different from each other and each represent a hydrogen atom, an alkyl group, an alkyl chain having a hydroxy group, an alkyl chain having an ether group, an alkyl chain having an ester group, or a benzyl group optionally having a substituent. R₂ and R₃ are identical to or different from each other and each represent a hydrogen atom, a hydroxyl group, a halogen, an alkyl group, an alkenyl group, an alkynyl group, an alkoxy group, an alkylsulfonyl group, or a phenyl group. Z represents a sulfur atom, an oxygen atom, or a carbon atom to which a methyl group has been bound. n represents 0, 1, 2, or 3. X⁻ represents an anion.

In the present embodiment, the alkyl group may be in the form of a straight chain or a branched chain. In the above formula (I), in a case where one of R₁ and R₄ is any of alkyl groups having 6 to 18 carbon atoms, the other one of R₁ and R₄ is preferably a hydrogen atom or an alkyl group having carbon atoms the number of which is smaller than 6. Among the alkyl groups having 6 to 18 carbon atoms, an alkyl group having 6, 8, or 10 carbon atoms is preferable.

Examples of the substituent of the benzyl group that can represent R₁ and R₄ in the above formula (I) include: alkyl groups having 1 to 20 carbon atoms; alkenyl groups having 2 to 20 carbon atoms; and alkynyl groups having 2 to 20 carbon atoms. Among these substituents, a methyl group and an ethyl group are particularly preferable.

Examples of the alkenyl group that can represent R₂ and R₃ in the above formula (I) include alkenyl groups having 2 to 20 carbon atoms. Examples of the alkoxy group that can represent R₂ and R₃ in the above formula (I) include alkoxy groups having 1 to 20 carbon atoms. Among these alkoxy groups, a methoxy group and an ethoxy group are particularly preferable.

Examples of the anion X⁻ in the above formula (I) include: halogen ions such as F⁻, Cl⁻, Br⁻, and I⁻; CF₃SO₃⁻; BF₄⁻; and the like.

As the fluorescent dye in the first reagent, one of these types of fluorescent dyes may be used, or two or more of these types of fluorescent dyes may be used.

The concentration of the fluorescent dye in the first reagent can be set as appropriate according to the type of the fluorescent dye, but is ordinarily 0.01 to 100 pg/pL and preferably 0.1 to 10 pg/µL. For example, in a case where a fluorescent dye represented by the above formula (I) is used as a fluorescent dye of the first reagent, the concentration of the fluorescent dye in the first reagent is preferably 0.2 to 0.6 pg/µL and more preferably 0.3 to 0.5 pg/µL.

The first reagent can be obtained by dissolving the above fluorescent dye in an appropriate solvent so as to attain the above concentration. The solvent is not particularly limited as long as the above fluorescent dye can be dissolved therein. Examples of the solvent include water, an organic solvent, and a mixture thereof. Examples of the organic solvent include alcohols, ethylene glycol, dimethyl sulfoxide (DMSO), and the like. The fluorescent dye might have poor preservation stability in an aqueous solution, and thus, is preferably dissolved in the organic solvent.

Alternatively, a commercially available staining reagent for classifying white blood cells may be used as the first reagent. Examples of such a staining reagent include Fluorocell WDF manufactured by Sysmex Corporation.

The second reagent contains a surfactant for hemolyzing red blood cells and damaging the cell membranes of white blood cells to such an extent that the fluorescent dye in the above first reagent can be transmitted through the cell membranes. For example, the second reagent contains at least one of or a combination of a cationic surfactant, a nonionic surfactant, and an anionic surfactant. The second reagent preferably further contains an aromatic organic acid having a concentration of 20 mM or higher and 50 mM or lower. Here, in a case where the concentration of the aromatic organic acid in the second reagent is 20 mM or higher and lower than 30 mM, the second reagent has a pH of preferably 5.5 or larger and 6.4 or smaller. Meanwhile, in a case where the concentration of the aromatic organic acid in the second reagent is 30 mM or higher and 50 mM or lower, the second reagent has a pH of preferably 5.5 or larger and 7.0 or smaller.

In the present embodiment, in the case where the concentration of the aromatic organic acid in the second reagent is 20 mM or higher and lower than 30 mM, the second reagent has a pH of preferably 5.5 or larger and 6.4 or smaller and more preferably 5.5 or larger and 6.2 or smaller. Meanwhile, in the case where the concentration of the aromatic organic acid in the second reagent is 30 mM or higher and 50 mM or lower and is preferably 40 mM or higher and 50 mM or lower, the second reagent has a pH of preferably 5.5 or larger and 7.0 or smaller. In the case where the concentration of the aromatic organic acid in the second reagent is 40 mM or higher and 50 mM or lower, the second reagent has a pH of further preferably 5.5 or larger and 6.2 or smaller.

In the present embodiment, the aromatic organic acid means either of: an acid having at least one aromatic ring in the molecule thereof; and a salt thereof. Examples of the aromatic organic acid include aromatic carboxylic acids, aromatic sulfonic acids, and the like. In the present embodiment, phthalic acid, benzoic acid, salicylic acid, hippuric acid, p-aminobenzenesulfonic acid, benzenesulfonic acid, and salts thereof are suitably usable. As the aromatic organic acid in the second reagent, one of these types of aromatic organic acids may be used, or two or more of these types of aromatic organic acids may be used. In a case where the second reagent contains two or more of these types of aromatic organic acids, the total concentration of the aromatic organic acids may be 20 mM or higher and 50 mM or lower.

As the cationic surfactant, it is possible to use, for example, a quaternary-ammonium-salt-type surfactant or a pyridinium-salt-type surfactant.

Examples of the quaternary-ammonium-salt-type surfactant include a surfactant having a total of 9 to 30 carbon atoms, the surfactant being represented by the following formula (II).

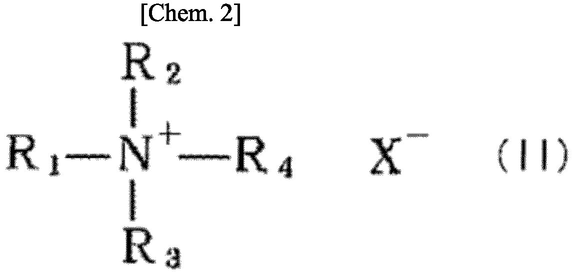

In the above formula (II), R₁ represents an alkyl group or an alkenyl group having 6 to 18 carbon atoms. R₂ and R₃ are identical to or different from each other and each represent an alkyl group or an alkenyl group having 1 to 4 carbon atoms. R₄ represents an alkyl group or an alkenyl group having 1 to 4 carbon atoms, or a benzyl group. X⁻ represents a halogen atom.

As R₁ in the above formula (II), an alkyl group or an alkenyl group having 6, 8, 10, 12, or 14 carbon atoms is preferable, and a straight-chain alkyl group is particularly preferable. More specific examples of R₁ include an octyl group, a decyl group, and a dodecyl group. As R₂ and R₃, a methyl group, an ethyl group, and a propyl group are preferable. As R₄, a methyl group, an ethyl group, and a propyl group are preferable.

Examples of the pyridinium-salt-type surfactant include a surfactant represented by the following formula (III).

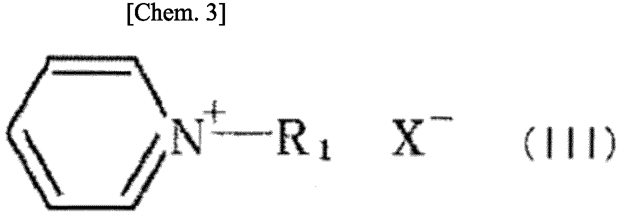

In the above formula (III), R₁ represents an alkyl group or an alkenyl group having 6 to 18 carbon atoms, and X⁻ represents a halogen atom.

As R₁ in the above formula (III), an alkyl group or an alkenyl group having 6, 8, 10, 12, or 14 carbon atoms is preferable, and a straight-chain alkyl group is particularly preferable. More specific examples of R₁ include an octyl group, a decyl group, and a dodecyl group.

The concentration of the cationic surfactant in the second reagent can be adjusted as appropriate according to the type of the surfactant, but is ordinarily 10 to 10000 ppm and preferably 100 to 1000 ppm.

As the nonionic surfactant, a polyoxyethylene-based nonionic surfactant represented by the following formula (VI) is preferable.

R₁-R₂-(CH₂CH₂O)ₙ-H ··· (VI)

In the above formula (VI), R₁ represents an alkyl group, an alkenyl group, or an alkynyl group having 8 to 25 carbon atoms, and R₂ represents an oxygen atom, -COO-, or a group represented by the following formula. 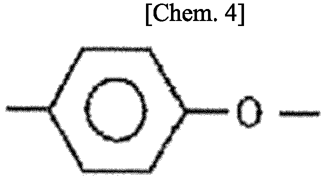 n represents an integer of 10 to 50.

Specific examples of the above nonionic surfactant include polyoxyethylene alkylethers, polyoxyethylene sterols, polyoxyethylene castor oils, polyoxyethylene sorbitol fatty acid esters, polyoxyethylene alkylamines, polyoxyethylene polyoxypropylene alkyl ethers, and the like.

The concentration of the nonionic surfactant in the second reagent is ordinarily 10 to 100000 ppm, preferably 100 to 10000 ppm, and more preferably 1000 to 5000 ppm.

The second reagent may contain a buffer for maintaining the pH thereof at a fixed value. Examples of such a buffer include citric acid salts, HEPES, phosphoric acid salts, and the like. The above aromatic organic acid sometimes exhibits a buffering effect. In the case of using such an aromatic organic acid, the buffer may be or does not have to be added to the second reagent.

The osmotic pressure of the second reagent is not particularly limited, but is preferably 20 to 150 mOsm/kg from the viewpoint of efficiently hemolyzing red blood cells.

The second reagent can be obtained by: dissolving the above surfactant, the above aromatic organic acid or a salt thereof, and, as necessary, the above buffer in an appropriate solvent so as to attain the above concentration of the aromatic organic acid; and adjusting the pH of the resultant solution by using NaOH, HCl, or the like. The solvent is not particularly limited as long as the above components can be dissolved therein. Examples of the solvent include water, an organic solvent, and a mixture thereof. Examples of the organic solvent include alcohols, methanol, ethylene glycol, DMSO, and the like.

Alternatively, a commercially available hemolyzing reagent for classifying white blood cells may be used as the second reagent. Examples of such a hemolyzing reagent include Lysercell WDF and Lysercell WDF II manufactured by Sysmex Corporation.

Owing to the second reagent composed as described above, red blood cells are hemolyzed, and the cell membranes of white blood cells are damaged to such an extent that the fluorescent dye in the first reagent can pass through the cell membranes. In addition, owing to the first reagent composed as described above, the nucleic acids of the white blood cells having the cell membranes damaged by the second reagent are stained. Consequently, it becomes possible for the optical detector D to detect four subclasses of white blood cells.

The detection part 26 is connected via a tube to the reagent container 253 containing the sheath liquid (diluent). In addition, the detection part 26 is connected to the compressor (not shown) and can obtain a portion of the sheath liquid (diluent) from the reagent container 253 by means of a pressure generated by the compressor. The obtained portion of the sheath liquid is used in the optical detector D.

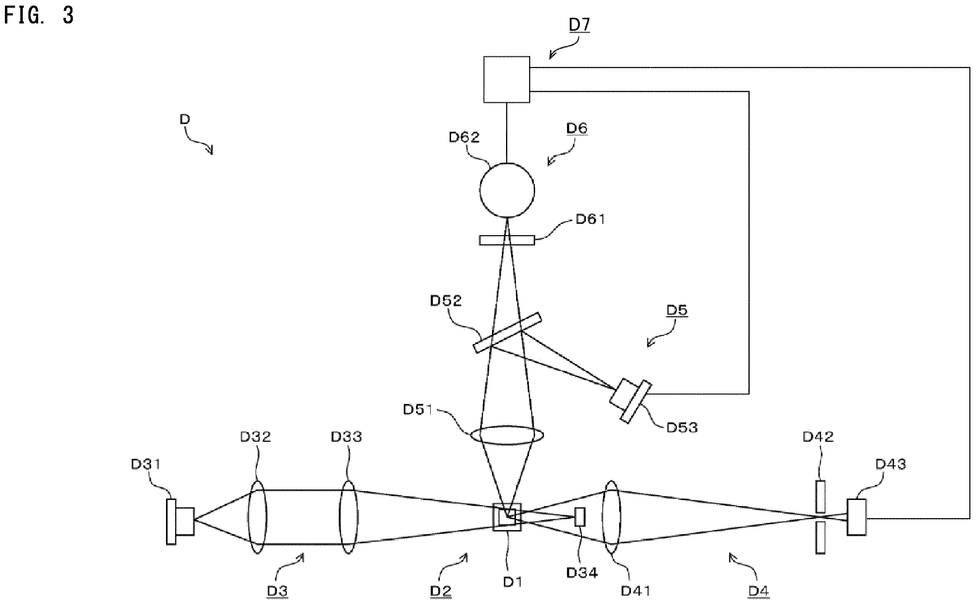

FIG. 3 shows a configuration of the optical detector. The optical detector D employs flow cytometry in which a semiconductor laser is used. The optical detector D includes a flow cell D1, a sheath flow system D2, a beam spot forming system D3, a forward scattered light receiving system D4, a side scattered light receiving system D5, a fluorescence receiving system D6, and a signal processing circuit D7.

The flow cell D1 is a member formed from a light-transmissive material so as to have a tubular shape. The sheath flow system D2 is configured to send the measurement sample into the flow cell D1 in a state where the measurement sample is wrapped by the sheath liquid, such that a liquid flow is generated in the flow cell D 1. The beam spot forming system D3 is configured such that light emitted from the semiconductor laser D31 as a light source is applied to the flow cell D1 through a collimator lens D32 and a condenser lens D33. Consequently, the laser light is applied to blood cells contained in the liquid flow passing through the inside of the flow cell D1. In addition, the beam spot forming system D3 further includes a beam stopper D34.

The forward scattered light receiving system D4 is configured to: condense, by a forward condenser lens D41, a light (forward scattered light) scattered forward by each of the blood cells to which the laser light has been applied; and receive, by a photodiode D43, the light having passed through a pinhole D42. The photodiode D43 outputs an electric signal having a magnitude based on the intensity of the received light. While the blood cell is passing through a laser light application region in the flow cell D1, the signal processing circuit D7 samples the electric signal, which has been outputted from the photodiode D43, a plurality of times so as to convert the electric signal into digital signals. The signal processing circuit D7 transmits the digital signals to the information processing unit 4. Each of the digital signals (waveform signals) is a result of measurement of the received forward scattered light and indicates a forward scattered light signal.

The side scattered light receiving system D5 is configured to: condense, by a side condenser lens D51, a light (side scattered light) scattered to the side by each of the blood cells to which the laser light has been applied; reflect the side scattered light by a dichroic mirror D52; and receive the reflected side scattered light by a photodiode D53. The photodiode D53 outputs an electric signal having a magnitude based on the intensity of the received light. While the blood cell is passing through the laser light application region in the flow cell D1, the signal processing circuit D7 samples the electric signal, which has been outputted from the photodiode D53, a plurality of times so as to convert the electric signal into digital signals. The signal processing circuit D7 transmits the digital signals to the information processing unit 4. Each of the digital signals (waveform signals) is a result of measurement of the received side scattered light and indicates a side scattered light signal.

Light scattering is a phenomenon that, when a particle such as a blood cell is present as an obstacle in the advancing direction of light, occurs owing to change in the advancing direction of the light by the particle. By detecting these scattered lights, information about the size and the internal structure of the particle can be obtained. In particular, information about the size of the particle (blood cell) can be obtained from the forward scattered light. Specifically, a higher forward scattered light intensity indicates that the size of the cell (e.g., white blood cell) is larger. Meanwhile, information about the inside of the particle can be obtained from the side scattered light. When the laser light is applied to a blood cell particle, the side scattered light intensity is dependent on the internal complexity of the cell (the shape and size of the nucleus thereof, the density of the cell, and the amount of granules of the cell). For example, a higher side scattered light intensity indicates that: the density of the cell (blood cell) is higher; and the amount of the granules contained in the cell (blood cell) is larger. Furthermore, a higher side scattered light intensity indicates that: the shape of the nucleus is more complicated; and the nucleus is segmented to a larger extent.

The fluorescence receiving system D6 is configured to receive a fluorescence by a photodiode D62 as an avalanche photodiode through the dichroic mirror D52 and a spectral filter D61, the fluorescence having been emitted as a result of applying the laser light to each of the blood cells of which the nucleic acids have been stained. The photodiode D62 outputs an electric signal having a magnitude based on the intensity of the received light. While the blood cell is passing through the laser light application region in the flow cell D1, the signal processing circuit D7 samples the electric signal, which has been outputted from the photodiode D62, a plurality of times so as to convert the electric signal into digital signals. The signal processing circuit D7 transmits the digital signals to the information processing unit 4. Each of the digital signals (waveform signals) is a result of measurement of the received fluorescence and indicates a fluorescence signal. Alternatively, the photodiode D62 does not have to be an avalanche photodiode and may be, for example, a photomultiplier.

When light is applied to a blood cell of which the nucleic acid has been stained with a fluorescent substance, the fluorescent substance emits a fluorescence. As the amount of the stained nucleic acid becomes larger, the intensity of the fluorescence becomes higher. Therefore, measurement of fluorescence signals makes it possible to classify blood cells on the basis of the difference among the amounts of the nucleic acids contained in the blood cells.

With reference back to FIG. 2, the results of the measurement including the forward scattered light signals, the side scattered light signals, and the fluorescence signals measured by the optical detector D are transmitted to the information processing unit 4.

As described above, the measurement unit 2 functions as: a preparation part which prepares a measurement sample containing a blood specimen and the fluorescent dye that stains white blood cells contained in the blood specimen; and a measurement part which applies light to the measurement sample, detects fluorescence from the white blood cells contained in the measurement sample to which the light has been applied, and measures fluorescence signals on the basis of the detected fluorescence.

### (Information Processing Unit)

FIG. 4 shows a configuration of the information processing unit. The information processing unit 4 is configured to include a computer and is composed of the main body 40, the input part 41, and the output part 42. The input part 41 and the output part 42 are as described above, and thus, will not be described below.

The main body 40 includes a CPU 401, a ROM 402, a RAM 403, a storage 404, a read-out device 405, an image output interface 406, an input/output interface 407, and a communication interface 408.

The CPU 401 executes a computer program stored in the ROM 402 and a computer program loaded to the RAM 403. The RAM 403 is used for reading out computer programs stored in the ROM 402 and the storage 404. In addition, the RAM 403 is also used as a work area for the CPU 401 at the time of executing these computer programs.

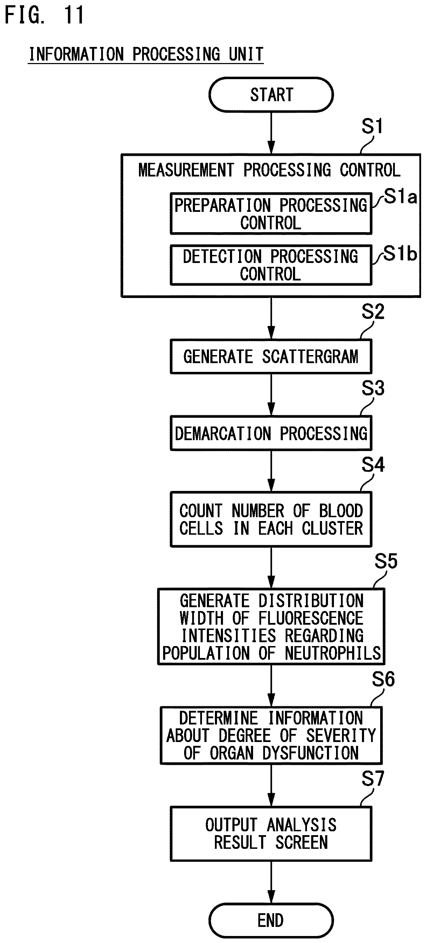

The storage 404 is a solid-state drive (SSD), a hard disk drive (HDD), or a combination thereof. The storage 404 stores therein: an operating system (OS); a computer program to be executed by the CPU 401; and data for use in execution of the computer program. In addition, the storage 404 stores therein a program 404a for causing the information processing unit 4 to execute a process shown in FIG. 11. The read-out device 405 is implemented by a CD drive, a DVD drive, or the like and can read out a computer program and data stored in a storage medium 405a. In a case where the above program 404a is stored in the storage medium 405a, the program 404a read out from the storage medium 405a by the read-out device 405 is stored in the storage 404.

The image output interface 406 outputs an image signal based on data to the output part 42, and the output part 42 displays an image on the basis of the data outputted from the image output interface 406. A user inputs an instruction through the input part 41, and the input/output interface 407 receives a signal inputted through the input part 41. The communication interface 408 is connected to the measurement unit 2, the transport unit 3, and the host computer 5, and the CPU 401 transmits/receives a control signal and data to/from these devices through the communication interface 408.

By the constituents 401 to 408, the main body 40 functions as: a controller for controlling the measurement unit 2 and the transport unit 3 through execution, by the CPU 401, of an instruction inputted by a user through the input part 41 and/or a computer program; and an information processing part through execution of a computer program by the CPU 401.

In other words, by the main body 40, the information processing unit 4 controls operations to be performed by the measurement unit 2 and the transport unit 3 and executes analysis on the basis of the results of the measurement (e.g., the forward scattered light signals, the side scattered light signals, and the fluorescence signals) received from the measurement unit 2. In this analysis, the white blood cells present in the blood specimen are classified into four subclasses, i.e., (1) lymphocytes (LYMPH), (2) monocytes (MONO), (3) a blood cell group composed of neutrophils (NEUT) and basophils (BASO), and (4) eosinophils (EO). A procedure for the classification will be described.

The information processing unit 4 extracts, from among the forward scattered light signals of each of the blood cells (the digital signals obtained by performing sampling a plurality of times while the blood cell is passing through the laser light application region in the flow cell D1) received from the measurement unit 2, a digital signal indicating a peak value of the blood cell and obtains the extracted digital signal as a forward scattered light intensity regarding the blood cell. Likewise, the information processing unit 4 extracts, from among the side scattered light signals of each of the blood cells, a digital signal indicating a peak value of the blood cell and obtains the extracted digital signal as a side scattered light intensity regarding the blood cell. Likewise, the information processing unit 4 extracts, from among the fluorescence signals of each of the blood cells, a digital signal indicating a peak value of the blood cell and obtains the extracted digital signal as a fluorescence intensity regarding the blood cell. Thereafter, the information processing unit 4 generates a scattergram. The scattergram is a distribution diagram in which each of the blood cells is plotted on the basis of the corresponding forward scattered light intensity, the corresponding side scattered light intensity, and the corresponding fluorescence intensity. The information processing unit 4 can generate: a three-dimensional scattergram having axes that indicate forward scattered light intensity, side scattered light intensity, and fluorescence intensity; a two-dimensional scattergram having axes that indicate side scattered light intensity and fluorescence intensity; and the like. The scattergram in the present embodiment is the two-dimensional scattergram.

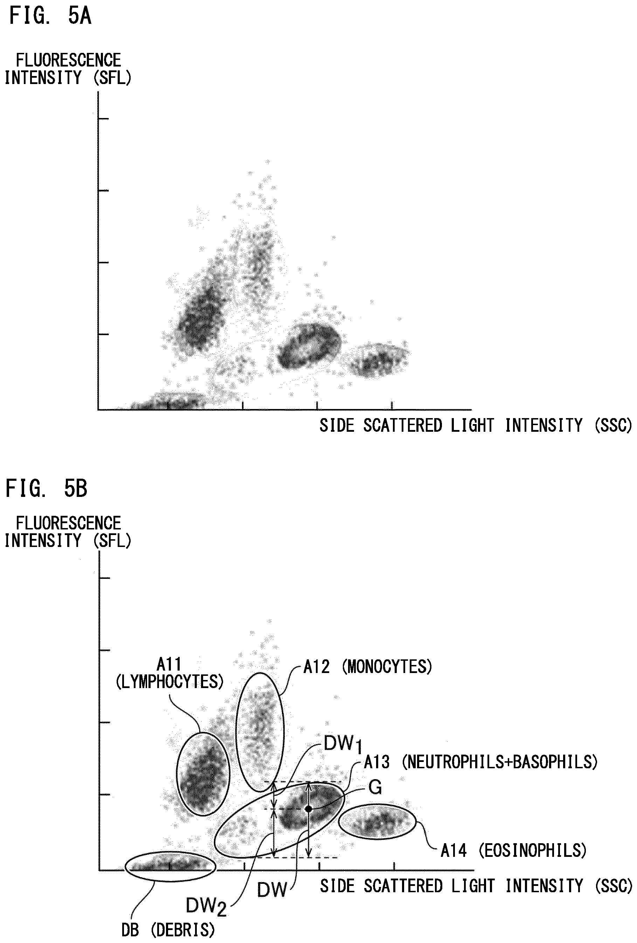

FIG. 5A shows an example of the scattergram. As shown in FIG. 5A, the scattergram in the present embodiment is generated by plotting each of the individual blood cells at a position on a two-dimensional diagram having a horizontal axis indicating side scattered light intensity (SSC) and a vertical axis indicating fluorescence intensity (SFL), the position being based on the corresponding side scattered light intensity and the corresponding fluorescence intensity regarding the blood cell that have been measured by the measurement unit 2.

Next, by the main body 40, the information processing unit 4 performs demarcation processing on the scattergram. The demarcation processing is processing for classifying each of the white blood cells on the scattergram into any of clusters such that the white blood cells are demarcated on the scattergram. A procedure and a method for the demarcation processing will be described.

FIG. 5B shows an example of the scattergram having been subjected to the demarcation processing. First, the information processing unit 4 eliminates, from among the dots plotted on the scattergram, dots corresponding to debris which are the hemolyzed red blood cells and the like. Specifically, the dots included in a preset (predetermined) region DB are eliminated. Subsequently, the information processing unit 4 classifies each of the dots on the scattergram that remain after excluding the blood cells in the region DB, into any of four clusters (populations) corresponding to the four subclasses of white blood cells. As shown in FIG. 5B, the four clusters are (1) a cluster A11 of lymphocytes (LYMPH), (2) a cluster A12 of monocytes (MONO), (3) a cluster A13 of the blood cell group composed of neutrophils (NEUT) and basophils (BASO), and (4) a cluster A14 of eosinophils (EO).

Specifically, the information processing unit 4 calculates, from the distances between each of the dots corresponding to the respective blood cells plotted on the scattergram and the positions of the centroids of the preset respective clusters, grades of membership of the corresponding blood cell to the respective clusters and sorts the blood cell into any of the clusters according to the grades of membership. Such a method for classifying blood cells is described in detail in US Patent No. 5555196, and US Patent No. 5555196 is incorporated herein by reference.

When each of the blood cells is classified into any of the four clusters corresponding to the four subclasses of white blood cells, the dots plotted on the scattergram are demarcated as in the clusters A11 to A14 shown in FIG. 5B.

The information processing unit 4 counts the number of the blood cells in each of the clusters. Specifically, by the main body 40, the information processing unit 4 counts the number of the dots included in each of the clusters A11 to A14 (the number of the blood cells belonging to each of the clusters). Consequently, the number of lymphocytes, the number of monocytes, the number of the blood cells in the blood cell group composed of neutrophils and basophils, and the number of eosinophils are obtained. In addition, by the main body 40, the information processing unit 4 counts the number of the white blood cells (i.e., the total number of the dots included in the clusters A11 to A 14) and calculates the proportions of the lymphocytes, the monocytes, the blood cells in the blood cell group composed of the neutrophils and the basophils, and the eosinophils to the white blood cells.

By the main body 40, the information processing unit 4 generates, on the basis of the fluorescence intensities regarding the blood cells belonging to the cluster A13, information about a distribution width of fluorescence intensities regarding a population of neutrophils. The population of neutrophils is the blood cell group composed of the neutrophils and the basophils and is a blood cell group included in the cluster A13. In general, much more neutrophils are contained in peripheral blood than basophils. Thus, the blood cell group in the cluster A13 can be interpreted as a population of neutrophils. Alternatively, the blood cells corresponding to the basophils may be excluded from the blood cell group in the cluster A13, and the remaining blood cell group may be regarded as a population of neutrophils.

The information about the distribution width of the fluorescence intensities regarding the population of neutrophils is an index indicating the distribution width of the fluorescence intensities regarding the population of neutrophils. The index indicating the distribution width of the fluorescence intensities regarding the population of neutrophils may be any of (1) a distance DW (see FIG. 5B) from the Y-axis upper limit to the Y-axis lower limit of the population of neutrophils based on the X axis passing through a centroid G (see FIG. 5B) of the population of neutrophils; (2) a distance DWi (see FIG. 5B) from the centroid G to the Y-axis upper limit of the population of neutrophils based on the X axis passing through the centroid G; (3) a distance DW₂ (see FIG. 5B) from the centroid G to the Y-axis lower limit of the population of neutrophils based on the X axis passing through the centroid G; or (4) the average of the distance DWi from the centroid G to the Y-axis upper limit of the population of neutrophils based on the X axis passing through the centroid G and the distance DW₂ from the centroid G to the Y-axis lower limit of the population of neutrophils based on the X axis passing through the centroid G. By the main body 40, the information processing unit 4 calculates the centroid G. Alternatively, the index indicating the distribution width of the fluorescence intensities regarding the population of neutrophils may be, irrespective of the centroid G, (5) a distance in the Y-axis direction between the blood cell at which the fluorescence intensity regarding the population of neutrophils has the maximum value and the blood cell at which the fluorescence intensity regarding the population of neutrophils has the minimum value. For example, as the information about the distribution width of the fluorescence intensities regarding the population of neutrophils, NE-WY outputted through the same method as that of the automatic multi-item blood cell analyzer XN series (XN-1000, XN-2000, and the like) manufactured by Sysmex Corporation may be used.

The present inventor has found that the distribution width of the fluorescence intensities regarding the population of neutrophils has a high correlation with sequential organ failure assessment (SOFA) score for use in evaluation of a degree of severity of an organ dysfunction due to an infection. In addition, the present inventor has, as a result of examining SOFA scores of patients in an initial stage of hospitalization, found that a distribution width of fluorescence intensities regarding a population of neutrophils of a patient suffering from a severe organ dysfunction due to an infection tends to be large. Experiments and analyses from which these findings have been obtained will be described.

The present inventor analyzed, through a method in which the blood cell analyzer 1 described above was used, blood specimens collected, on each day in the initial stage of hospitalization, from hospitalized patients suspected of suffering from sepsis and blood specimens collected from healthy individuals. In the analyses, blood specimens collected from 44 examples of hospitalized patients suspected of suffering from sepsis and 39 examples of healthy individuals were used. The hospitalized patients suspected of suffering from sepsis were categorized into: 23 examples of sepsis patients diagnosed as suffering from sepsis after being hospitalized; and 21 examples of non-sepsis patients who were suspected of suffering from sepsis but did not suffer from sepsis.

A selection criterion for hospitalized patients suspected of suffering from sepsis was as follows. That is, patients having fever (38°C or higher) and having qSOFA (quick SOFA) scores of 2 points or larger, were regarded as hospitalized patients suspected of suffering from sepsis. It is noted that patients to whom steroids were administered were expressly registered as having had the steroids administered. The qSOFA is composed of three items, i.e., respiratory frequency, mental state, and systolic blood pressure, and is a simpler sepsis screening method than SOFA. An exclusion criterion for hospitalized patients suspected of suffering from sepsis was as follows. That is, patients in a terminal stage of a chronic disease, patients suffering from neutropenia, dialysis patients, and patients to whom ACTEMRA (registered trademark) (humanized anti-human IL-6 receptor monoclonal antibody) and a TNFα inhibitor were administered, were not regarded as hospitalized patients suspected of suffering from sepsis.

A selection criterion for healthy individuals was as follows. That is, persons who did not have fever at the time of collecting a specimen therefrom and who were not suspected of suffering from any infection, persons who did not contract any chronic inflammatory disease, persons who had normal coagulability, and persons who did not orally take any anticoagulant, were regarded as healthy individuals. No exclusion criterion for healthy individuals was set.

Specifically, from each of the hospitalized patients suspected of suffering from sepsis, the present inventor collected a blood specimen on each of the initial day (zeroth day), the first day, and the third day after hospitalization, which were an initial stage of hospitalization. Meanwhile, from each of the healthy individuals, the present inventor collected a blood specimen only once. Furthermore, a test for obtaining SOFA scores was conducted on each of the hospitalized patients, to obtain a SOFA score on each of the days (the zeroth day, the first day, and the third day). In addition, the present inventor caused the measurement unit 2 to, through the method of the measurement unit 2, prepare measurement samples on each day regarding the hospitalized patients and measurement samples regarding the healthy individuals by using the collected blood specimens and measure side scattered light signals and fluorescence signals. Then, the present inventor caused the information processing unit 4 to, through the method of the information processing unit 4, calculate white blood cell-related parameters including information (NE-WY) about a distribution width of fluorescence intensities regarding a population of neutrophils of each of the measurement samples. The automatic multi-item blood cell analyzer XN-2000 manufactured by Sysmex Corporation was used for the preparation, the measurement of the side scattered light signals and the fluorescence signals, and the calculation of the white blood cell-related parameters including the NE-WY.

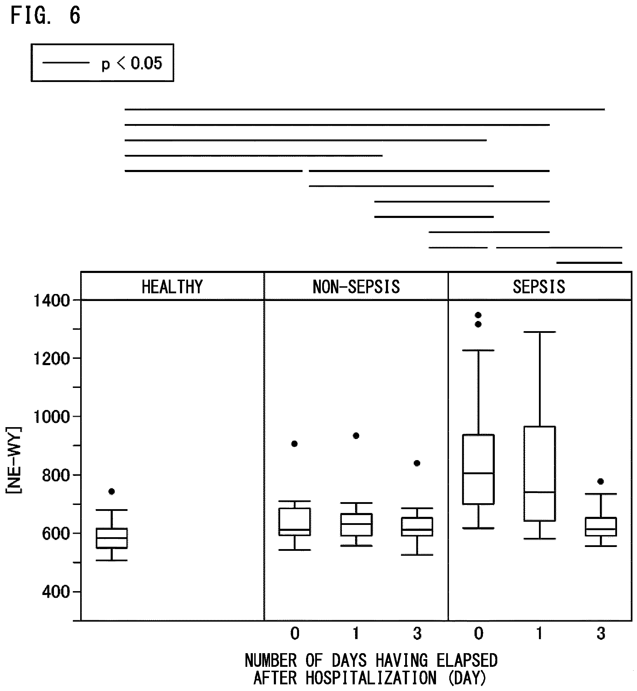

FIG. 6 shows graphs of information (NE-WY) about distribution widths of fluorescence intensities regarding populations of neutrophils of the hospitalized patients who suffered from sepsis (sepsis patients) and the hospitalized patients who did not suffer from sepsis (non-sepsis patients) with respect to the number of days having elapsed after hospitalization, and a graph of information (NE-WY) about distribution widths of fluorescence intensities regarding populations of neutrophils of the healthy individuals. Each of the graphs in FIG. 6 is a boxplot. Each of the lateral lines shown on the upper side relative to the graphs in FIG. 6 indicates two groups between which a p-value at the time of testing the difference between the data groups through the Steel-Dwass method, is smaller than 0.05. For example, the first lateral line from the top indicates that the p-value between a data group of the NE-WY about the healthy individuals and a data group of the NE-WY about the sepsis patients on the third day after hospitalization at the time of conducting a test through this method, is smaller than 0.05. The second lateral line from the top indicates that the p-value between the data group of the NE-WY about the healthy individuals and a data group of the NE-WY about the sepsis patients on the first day after hospitalization, is smaller than 0.05. The third lateral line from the top indicates that the p-value between the data group of the NE-WY about the healthy individuals and a data group of the NE-WY about the sepsis patients on the zeroth day after hospitalization, is smaller than 0.05. The sixth lateral line from the top indicates that the p-value between a data group of the NE-WY about the non-sepsis patients on the zeroth day after hospitalization and the data group of the NE-WY about the sepsis patients on the zeroth day after hospitalization, is smaller than 0.05. The seventh lateral line from the top indicates that the p-value between a data group of the NE-WY about the non-sepsis patients on the first day after hospitalization and the data group of the NE-WY about the sepsis patients on the first day after hospitalization, is smaller than 0.05. As shown in FIG. 6, it has been found that the NE-WY about the group of sepsis patients on the zeroth day after hospitalization, is significantly larger than the NE-WY about the group of healthy individuals and the NE-WY about the group of non-sepsis patients on the zeroth day after hospitalization. Likewise, it has been found that the NE-WY about the group of sepsis patients on the first day after hospitalization, is significantly larger than the NE-WY about the group of healthy individuals and the NE-WY about the group of non-sepsis patients on the first day after hospitalization. In this manner, it has been found that the NE-WY about the groups of sepsis patients in the initial stage of hospitalization, i.e., on the zeroth day and the first day after hospitalization, are significantly larger than the NE-WY about each of the group of healthy individuals and the groups of non-sepsis patients in the initial stage of hospitalization. Regarding the fluorescence intensity, a higher fluorescence intensity indicates that the amount of the nucleic acid is larger. Thus, the fact that the distribution width of the fluorescence intensities regarding the population of neutrophils is large is inferred to indicate that neutrophils having large amounts of nucleic acids and neutrophils having small amounts of nucleic acids are present, i.e., the neutrophils are diverse. The reason why sepsis leads to a diversity of neutrophils is considered to be, but is not limited to, the following reason. That is, in sepsis, immature neutrophils are mobilized into peripheral blood. The peripheral blood contains the mobilized immature neutrophils and neutrophils that are dead owing to apoptosis, necrosis, or the like. The amounts of the nucleic acids contained in the immature neutrophils are large, whereas the amounts of the nucleic acids contained in the dead neutrophils are small. Consequently, sepsis is considered to lead to increase in the distribution width of the fluorescence intensities regarding the population of neutrophils.

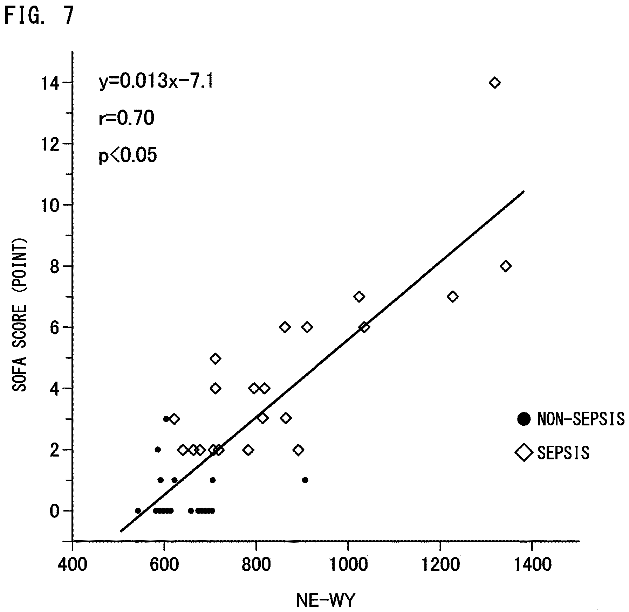

FIG. 7 shows a correlation between the information (NE-WY) about the distribution widths of the fluorescence intensities regarding the populations of neutrophils of the hospitalized patients (the sepsis patients and the non-sepsis patients) on the zeroth day after hospitalization and the SOFA score on said day. In FIG. 7, the white dots are data of the sepsis patients, and the black dots are data of the non-sepsis patients. A p-value at the time of checking the significance of the regression through an F-test, is smaller than 0.05. A Spearman's rank correlation coefficient r indicating the correlation between the NE-WY and the SOFA score is 0.70, and thus the correlation therebetween has been found to be high. This has led to obtainment of the finding that, from the information about the distribution width of the fluorescence intensities regarding the population of neutrophils, a corresponding SOFA score can be estimated. In addition, the present inventor has calculated a regression expression from the data of the sepsis patients and the non-sepsis patients shown in FIG. 7. The regression expression is y=0.013x-7.1. In this expression, the NE-WY is set as an x axis, and the SOFA score is set as a y axis.

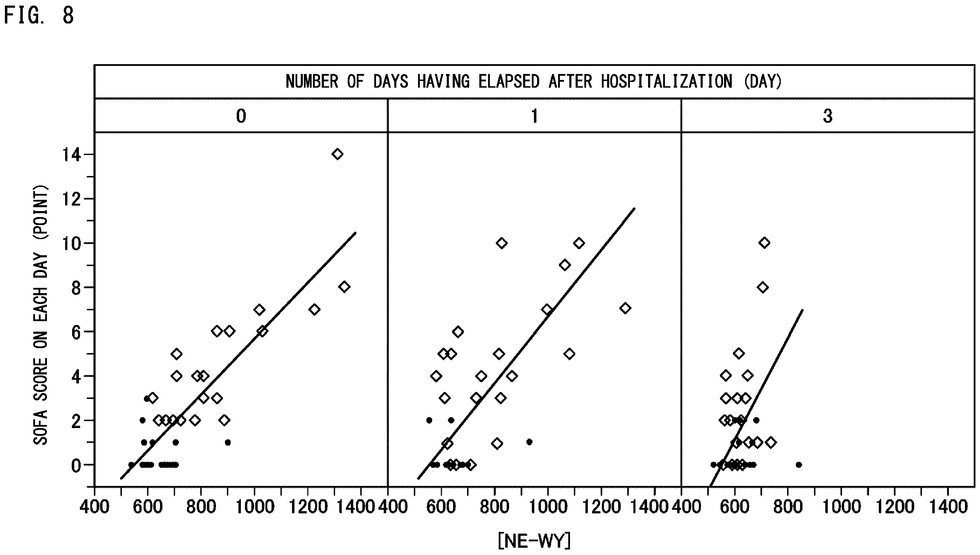

FIG. 8 shows correlations each of which is a correlation between the NE-WY on a corresponding one of the days and the SOFA score on said day. In FIG. 8, the leftmost graph is the same as the graph shown in FIG. 7. Each of the center graph and the rightmost graph in FIG. 8 is a graph indicating a correlation between the NE-WY about the hospitalized patients (the sepsis patients and the non-sepsis patients) on the corresponding one of the first day after hospitalization and the third day after hospitalization and the SOFA score on said day. In each of the graphs, the white dots are data of the sepsis patients, and the black dots are data of the non-sepsis patients. Spearman's rank correlation coefficients r each indicating the correlation between the NE-WY on the corresponding one of the initial day of (zeroth day after) hospitalization, the first day after hospitalization, and the third day after hospitalization and the SOFA score on said day, are respectively 0.70, 0.54, and 0.27. The correlation on the initial day of (zeroth day after) hospitalization has been found to be highest, and the correlation on the first day after hospitalization has been found to be second highest. In addition, it has been found that the correlation decreases day by day after hospitalization. The reason why the correlation decreases day by day is considered to be because a therapy is performed through administration of medication so as to suppress the infection from the initial day of hospitalization, so that the infection subsides according to the therapy. In addition, the present inventor has calculated regression expressions from the data of the sepsis patients and the non-sepsis patients on the respective days shown in FIG. 8. The regression expressions for the initial day of (zeroth day after) hospitalization, the first day after hospitalization, and the third day after hospitalization, are respectively y=0.013x-7.1, y=0.015x-8.3, and y=0.023x-12.1. In each of these expressions, the NE-WY is set as an x axis, and the SOFA score on the corresponding day is set as a y axis.

Now, description is returned to the information processing unit 4. By the main body 40, the information processing unit 4 determines information about a degree of severity of an organ dysfunction due to an infection on the basis of the information about the distribution width of the fluorescence intensities regarding the population of neutrophils. The information about the degree of severity of the organ dysfunction due to the infection is, for example, information about the degree of severity of the organ dysfunction suffered by the subject who has contracted the infection. Specifically, the information about the degree of severity of the organ dysfunction due to the infection includes: flag information indicating that the subject from whom the blood specimen has been collected is suspected of suffering from sepsis; and/or a range of an estimated score indicating the degree of severity of the organ dysfunction due to the infection. The estimated score indicating the degree of severity of the organ dysfunction due to the infection is an estimated value of the SOFA score (hereinafter, simply referred to also as "estimated SOFA score").

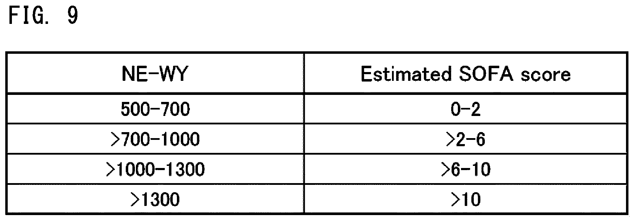

Specifically, the storage 404 stores therein a first conversion table in which the information about the distribution width of the fluorescence intensities regarding the population of neutrophils and the estimated SOFA score are associated with each other. FIG. 9 shows an example of the first conversion table. In the example in FIG. 9, an estimated SOFA score of 0 or larger and smaller than 2, an estimated SOFA score of 2 or larger and smaller than 6, an estimated SOFA score of 6 or larger and smaller than 10, and an estimated SOFA score of 10 or larger are respectively associated with a case where the information (NE-WY) about the distribution width of the fluorescence intensities regarding the population of neutrophils is 500 or larger and smaller than 700, a case where the NE-WY is 700 or larger and smaller than 1000, a case where the NE-WY is 1000 or larger and smaller than 1300, and a case where the NE-WY is 1300 or larger. In this manner, the first conversion table is such that, as the distribution width of the fluorescence intensities regarding the population of neutrophils becomes larger, the estimated score indicating the degree of severity of the organ dysfunction due to the infection becomes larger. The first conversion table can be created by obtaining a regression expression based on the data, of the sepsis patients and the non-sepsis patients, obtained through a method described in relation to the above experimenting method. For example, the first conversion table in FIG. 9 is created on the basis of the regression expression shown in FIG. 7.

By the main body 40, the information processing unit 4 determines, on the basis of the first conversion table and the generated information about the distribution width of the fluorescence intensities regarding the population of neutrophils, a range of an estimated score indicating the degree of severity of the organ dysfunction due to the infection, the range corresponding to the generated information about the distribution width of the fluorescence intensities regarding the population of neutrophils. For example, in a case where the NE-WY is 600, the information processing unit 4 determines that the range of the estimated score is 0 or larger and smaller than 2.

The information processing unit 4 determines flag information on the basis of the determined range of the estimated score. The flag information is a text indicating that the subject from whom the blood specimen has been collected is suspected of suffering from sepsis. The flag information is stored in the storage 404. The text is, for example, "Sepsis?". Specifically, the information processing unit 4 obtains the flag information in a case where, for example, the determined range of the estimated score is any of 2 or larger and smaller than 6, 6 or larger and smaller than 10, and 10 or larger.

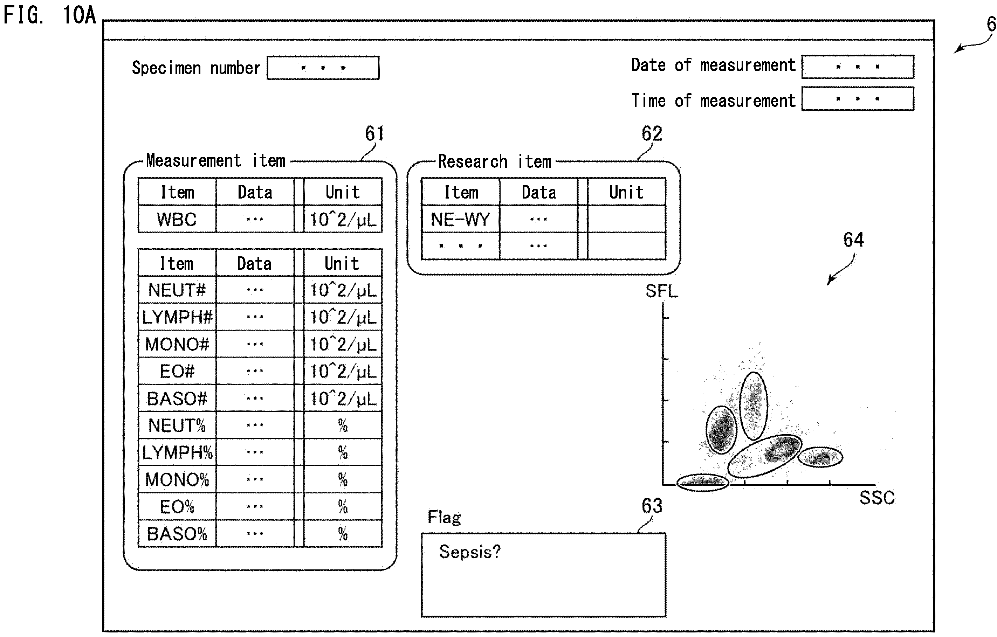

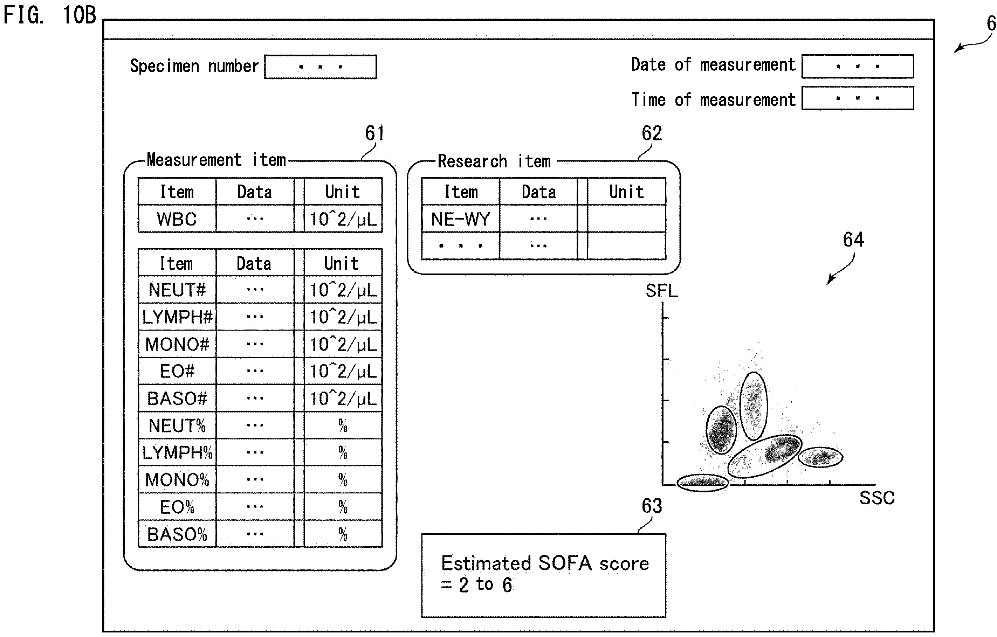

The information processing unit 4 causes the output part 42 to display the result of the analysis regarding the blood specimen. FIG. 10A shows an example of an analysis result screen regarding the blood specimen. FIG. 10B shows another example of the analysis result screen regarding the blood specimen. The main body 40 causes the output part 42 to display an analysis result screen 6 shown in either of FIG. 10A and FIG. 10B. The analysis result screen 6 includes the specimen number of the blood specimen, the date and time of the measurement, and regions 61 to 64. The region 61 is a region in which the number of the neutrophils (NEUT), the number of the lymphocytes (LYMPH), the number of the monocytes (MONO), the number of the eosinophils (EO), and the number of the basophils (BASO), and the proportion of each of the numbers to the number of the white blood cells (WBC), are displayed together with the number of the white blood cells. The region 62 is a region in which data of the index indicating the distribution width of the fluorescence intensities regarding the population of neutrophils is displayed. Here, data of the NE-WY is displayed in the region 62. The region 63 is a region in which the information about the degree of severity of the organ dysfunction due to the infection is displayed. In the region 63, the flag information indicating that the subject from whom the blood specimen has been collected is suspected of suffering from sepsis and/or the range of the estimated score indicating the degree of severity of the organ dysfunction due to the infection is displayed. In the example shown in FIG. 10A, the text "Sepsis?" is displayed as the flag information indicating that the subject is suspected of suffering from sepsis. In a case where the range of the estimated score is 0 or larger and smaller than 2, the flag information about the degree of severity of the organ dysfunction due to the infection is not displayed. In the example shown in FIG. 10B, the range (in the example in FIG. 10B, 2 or larger and smaller than 6) of the estimated SOFA score indicating the degree of severity of the organ dysfunction due to the infection is displayed. In the examples shown in FIG. 10A and FIG. 10B, the flag information and the range of the estimated score are respectively displayed alone in the region 63. Alternatively, both the flag information and the range of the estimated score may be displayed in the region 63. In the region 64, a scattergram generated by the information processing unit 4 is displayed. This scattergram is a two-dimensional scattergram having a horizontal axis indicating side scattered light intensity and a vertical axis indicating fluorescence intensity.

As described above, the information processing unit 4 functions as: an information processing part which generates, on the basis of the measured fluorescence signals, information about a distribution width of fluorescence intensities regarding a population of neutrophils contained in the measurement sample and determines information about a degree of severity of an organ dysfunction due to an infection on the basis of the information about the distribution width of the fluorescence intensities regarding the population of neutrophils; and an output part which outputs the information about the degree of severity of the organ dysfunction due to the infection.

Next, operation by the information processing unit 4 will be described. FIG. 11 shows an example of a flowchart of operation by the information processing unit. It is assumed that the transport unit 3 has supplied a blood specimen to the measurement unit 2 through control by the information processing unit 4.

As shown in FIG. 11, by the CPU 401, the information processing unit 4 executes measurement processing control (S1). The measurement processing control is control for causing the measurement unit 2 to perform measurement processing and includes preparation processing control and detection processing control. First, the information processing unit 4 executes the preparation processing control (S1a). Specifically, the information processing unit 4 transmits a first control signal to the measurement unit 2, thereby causing the measurement unit 2 to perform preparation processing. The preparation processing is processing for, as described above, mixing the blood specimen, the first reagent, and the second reagent with one another in order to hemolyze red blood cells contained in the blood specimen and fluorescently stain the nucleic acids of white blood cells, and heating the resultant liquid mixture by the heater H, to produce a measurement sample.

Next, by the CPU 401, the information processing unit 4 executes the detection processing control (S1b). Specifically, the information processing unit 4 transmits a second control signal to the measurement unit 2, thereby causing the measurement unit 2 to perform detection processing. The detection processing is blood cell detection processing based on the measurement sample as described above. Specifically, the measurement unit 2 causes the measurement sample to flow through the flow cell D1 so as to generate a liquid flow and causes the optical detector D to measure a forward scattered light signal, a side scattered light signal, and a fluorescence signal regarding each of the blood cells, on the basis of the second control signal. The measurement unit 2 transmits each of the measured signals and the corresponding specimen number to the information processing unit 4.

By the CPU 401, the information processing unit 4 stores, in the storage 404, the forward scattered light signal, the side scattered light signal, and the fluorescence signal regarding each of the blood cells received from the measurement unit 2 such that these signals are associated with the specimen number received from the measurement unit 2. By the CPU 401, the information processing unit 4 generates a scattergram on the basis of the received side scattered light signals and fluorescence signals (S2) and executes demarcation processing on the generated scattergram (S3). The scattergram generation processing and the demarcation processing are executed through the method described with reference to FIG. 5A and FIG. 5B.

By the CPU 401, the information processing unit 4 counts the number of the white blood cells (WBC) contained in the measurement sample and the numbers of the blood cells included in the respective clusters A11 to A14, and calculates the proportions of the blood cells in the respective clusters to the white blood cells (S4). By the CPU 401, the information processing unit 4 stores, in the storage 404, the number of the white blood cells and the numbers of the blood cells in the respective clusters having been counted and the proportions having been calculated, such that these numbers and proportions are associated with the specimen number (S4).

By the CPU 401, the information processing unit 4 generates, on the basis of fluorescence intensities regarding the population of neutrophils, information about a distribution width of the fluorescence intensities regarding the population of neutrophils (S5). Specifically, the information about the distribution width of the fluorescence intensities regarding the population of neutrophils is NE-WY.

Then, by the CPU 401, the information processing unit 4 determines information about a degree of severity of an organ dysfunction due to an infection on the basis of the information about the distribution width of the fluorescence intensities regarding the population of neutrophils (S6). Specifically, by the CPU 401, the information processing unit 4 refers to the first conversion table stored in the storage 404 and determines a range of an estimated SOFA score indicating the degree of severity of the organ dysfunction due to the infection, the range corresponding to the NE-WY generated in S5. When, for example, the value of the NE-WY generated in step S5 is 500 or larger and smaller than 700, the information processing unit 4 determines that the range of the estimated SOFA score is 0 or larger and smaller than 2. Meanwhile, when, for example, the value of the NE-WY is 700 or larger and smaller than 1000, the information processing unit 4 determines that the range of the estimated SOFA score is 2 or larger and smaller than 6. At this time, when the determined range of the estimated SOFA score is any of 2 or larger and smaller than 6, 6 or larger and smaller than 10, and 10 or larger, the information processing unit 4 reads out, by the CPU 401, flag information from the storage 404. Then, by the CPU 401, the information processing unit 4 generates the analysis result screen 6 regarding the blood specimen including the information (in the present embodiment, the range of the estimated SOFA score) about the degree of severity of the organ dysfunction due to the infection and causes the output part 42 to output (display) the analysis result screen 6 (S7). In the above embodiment, the measurement unit 2 transmits fluorescence signals as waveform signals to the information processing unit 4, and the information processing unit 4 obtains fluorescence intensities on the basis of the fluorescence signals. Alternatively, the measurement unit 2 may obtain fluorescence intensities as fluorescence signals and transmit the fluorescence intensities to the information processing unit 4. In the above embodiment, each of the fluorescence intensities is the peak value among the fluorescence signals regarding the corresponding blood cell. Alternatively, the fluorescence intensity may be a statistical value such as the average value among the fluorescence signals. The information about the degree of severity of the organ dysfunction due to the infection may be outputted as text data or image data.

### [1-3. Actions and Effects of First Embodiment]

(1) The blood cell analyzer 1 of the present embodiment includes: a measurement part (measurement unit 2) which applies light to a measurement sample containing a blood specimen and a fluorescent dye that stains white blood cells contained in the blood specimen, detects fluorescence from the white blood cells contained in the measurement sample to which the light has been applied, and measures fluorescence signals on the basis of the detected fluorescence; an information processing part (main body 40) which generates, on the basis of the measured fluorescence signals, information about a distribution width of fluorescence intensities regarding a population of neutrophils contained in the measurement sample and determines information about a degree of severity of an organ dysfunction due to an infection on the basis of the information about the distribution width of the fluorescence intensities regarding the population of neutrophils; and an output part 42 which outputs the information about the degree of severity of the organ dysfunction due to the infection.
   Consequently, it is possible to provide a specimen analysis method and a specimen analyzer for performing assistance in a swift evaluation of a degree of severity of an organ dysfunction due to an infection, the specimen analysis method and the specimen analyzer enabling outputting of information having a high correlation with a score indicating the degree of severity of the organ dysfunction due to the infection.
(2) The information about the degree of severity of the organ dysfunction due to the infection includes flag information indicating that a subject from whom the blood specimen has been collected is suspected of suffering from sepsis. Consequently, it is possible to assist a doctor in diagnosing that the subject suffers from sepsis.
(3) The information about the degree of severity of the organ dysfunction due to the infection includes a range of an estimated score indicating the degree of severity of the organ dysfunction due to the infection. Consequently, it is possible to assist a doctor in determining a therapeutic strategy for the subject. For example, it is possible to assist a doctor in deciding to, as the values included in the range of the estimated score become larger, provide a more intensive therapy.
(4) The information about the distribution width of the fluorescence intensities regarding the population of neutrophils is an index indicating the distribution width of the fluorescence intensities regarding the population of neutrophils, and, as the index becomes larger, the estimated score indicating the degree of severity of the organ dysfunction due to the infection becomes larger. Consequently, it is possible to assist a doctor in deciding to, as the value of the estimated score becomes larger, provide a more intensive therapy.
(5) The blood cell analyzer 1 of the present embodiment further includes a preparation part (sample preparation part 25) which prepares the measurement sample. Consequently, analysis of the white blood cells contained in the blood specimen can be facilitated.
(6) The measurement part (measurement unit 2) further includes a flow cell D1 through which the measurement sample is caused to flow, and applies the light to the measurement sample flowing through the flow cell D1. Consequently, a scattered light signal and a fluorescence signal regarding each of the blood cells can be obtained.

### [2. Second Embodiment]

A blood cell analyzer according to a second embodiment will be described. Regarding the second embodiment, only features different from those in the first embodiment will be described, and the same features as those in the first embodiment will not be described.

The second embodiment differs from the first embodiment in terms of the method for obtaining the information about the degree of severity of the organ dysfunction due to the infection. In addition, information about the degree of severity of the organ dysfunction due to the infection according to the second embodiment includes an estimated score indicating the degree of severity of the organ dysfunction due to the infection. This estimated score is an estimated SOFA score.

Specifically, by the main body 40, the information processing unit 4 generates an estimated SOFA score from a regression expression in which the information (NE-WY) about the distribution width of the fluorescence intensities regarding the population of neutrophils and the estimated SOFA score are associated with each other. That is, in the second embodiment, a computer program including the regression expression is, instead of the first conversion table in the first embodiment, stored in the storage 404, and, by the main body 40, the information processing unit 4 executes the computer program including the regression expression, to generate an estimated SOFA score corresponding to the NE-WY. The regression expression is, for example, y=0.013x-7.1 shown in FIG. 7. Therefore, the regression expression is a linear equation that is positively sloped, and thus, as the NE-WY becomes larger, the estimated score indicating the degree of severity of the organ dysfunction due to the infection becomes larger. The regression expression may be calculated from pieces of data of sepsis patients and non-sepsis patients, the number of which is sufficient to attain reliability.

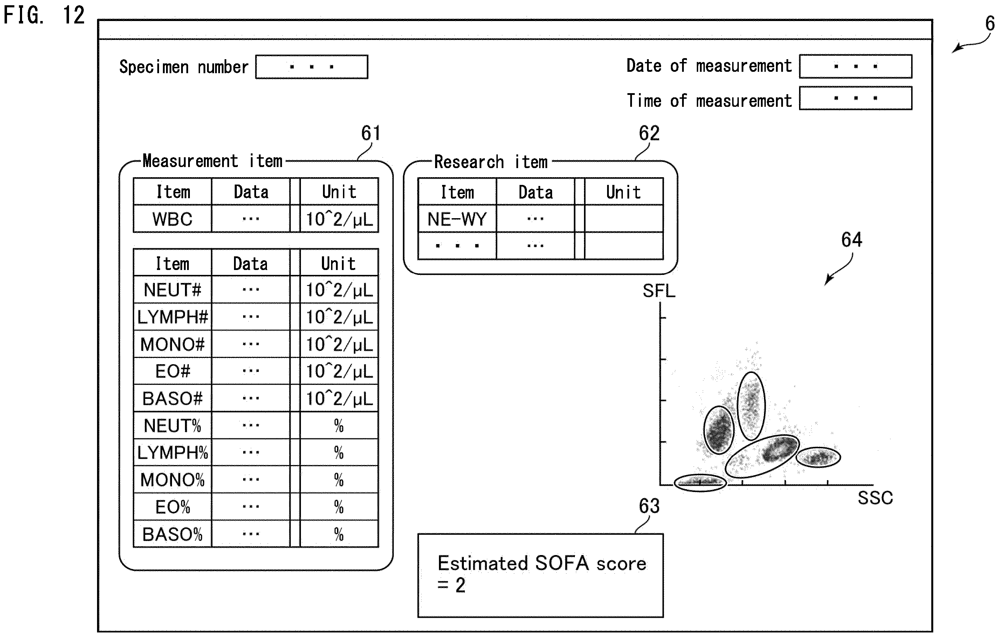

FIG. 12 shows an example of an analysis result screen regarding the blood specimen according to the second embodiment. As shown in FIG. 12, the information processing unit 4 causes the generated estimated SOFA score to be displayed in the region 63 of the analysis result screen 6. In the example shown in FIG. 12, it is indicated that the estimated SOFA score is 2.

In this manner, the information about the degree of severity of the organ dysfunction due to the infection includes the estimated score indicating the degree of severity of the organ dysfunction due to the infection, and in this case as well, it is possible to provide a specimen analysis method and a specimen analyzer for performing assistance in a swift evaluation of a degree of severity of an organ dysfunction due to an infection, the specimen analysis method and the specimen analyzer enabling outputting of information having a high correlation with a score indicating the degree of severity of the organ dysfunction due to the infection. For example, it is possible to assist a doctor in deciding to, as the value of the estimated score becomes larger, provide a more intensive therapy.

In addition, the information about the distribution width of the fluorescence intensities regarding the population of neutrophils is an index indicating the distribution width of the fluorescence intensities regarding the population of neutrophils, and, as the index becomes larger, the estimated score indicating the degree of severity of the organ dysfunction due to the infection becomes larger. Consequently, it is possible to assist a doctor in deciding to, as the value of the estimated score becomes larger, provide a more intensive therapy.

### [3. Third Embodiment]

A blood cell analyzer according to a third embodiment will be described. Regarding the third embodiment, only features different from those in the first embodiment will be described, and the same features as those in the first embodiment will not be described.

Information about the degree of severity of the organ dysfunction due to the infection according to the third embodiment differs from that according to the first embodiment. Specifically, the information about the degree of severity of the organ dysfunction due to the infection according to the third embodiment is flag information indicating, in a plurality of levels, that the subject from whom the blood specimen has been collected is suspected of suffering from sepsis.

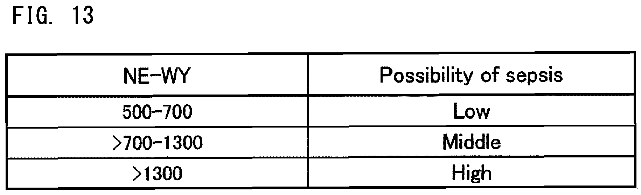

FIG. 13 shows an example of a second conversion table in which the NE-WY and the possibility of sepsis are associated with each other in a plurality of levels. As shown in FIG. 13, in the second conversion table, the possibilities of sepsis being "Low", "Middle", and "High" are respectively associated with a case where the NE-WY is 500 or larger and smaller than 700, a case where the NE-WY is 700 or larger and smaller than 1300, and a case where the NE-WY is 1300 or larger. In the third embodiment, the second conversion table is, instead of the first conversion table in the first embodiment, stored in the storage 404, and, by the main body 40, the information processing unit 4 determines, on the basis of the generated NE-WY and the second conversion table, the level of the possibility that the subject suffers from sepsis.

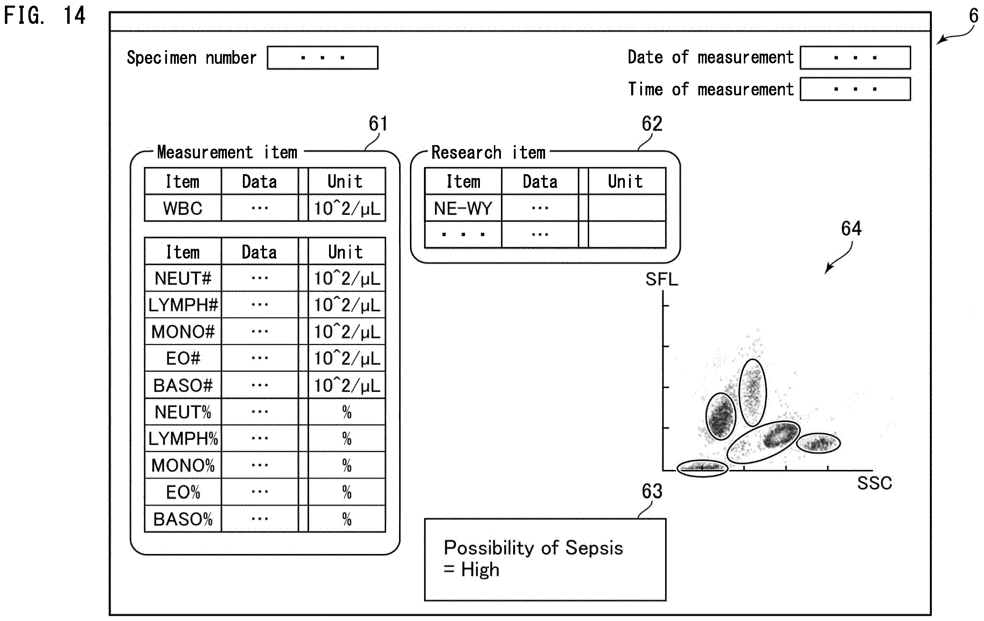

FIG. 14 shows an example of an analysis result screen regarding the blood specimen according to the third embodiment. As shown in FIG. 14, the information processing unit 4 causes the obtained level of the possibility of sepsis to be displayed as flag information in the region 63 of the analysis result screen 6. In the example shown in FIG. 14, it is indicated that the possibility of sepsis is "High".

In this manner, the information about the degree of severity of the organ dysfunction due to the infection is flag information indicating a level of the possibility of sepsis, and in this case as well, it is possible to provide a specimen analysis method and a specimen analyzer for performing assistance in a swift evaluation of a degree of severity of an organ dysfunction due to an infection, the specimen analysis method and the specimen analyzer enabling outputting of information having a high correlation with a score indicating the degree of severity of the organ dysfunction due to the infection. For example, it is possible to assist a doctor in deciding to, as the level indicating the degree of severity becomes higher, provide a more intensive therapy.

Although the possibility of sepsis as flag information is provided in three levels in the present embodiment, the possibility of sepsis may be provided in two levels, four levels, or five or more levels. Alternatively, the possibility of sepsis may be displayed as a probability.

### [4. Fourth Embodiment]

A blood cell analyzer according to a fourth embodiment will be described. Regarding the fourth embodiment, only features different from those in the first embodiment will be described, and the same features as those in the first embodiment will not be described.

Information about the degree of severity of the organ dysfunction due to the infection according to the fourth embodiment differs from that according to the first embodiment. Specifically, the information about the degree of severity of the organ dysfunction due to the infection according to the fourth embodiment includes, regarding flag information indicating that the subject from whom the blood specimen has been collected is suspected of suffering from sepsis, information indicating whether or not an estimated value of a difference between a SOFA score in an initial stage of hospitalization and a SOFA score in a healthy state is equal to or larger than a threshold value.

Specifically, a threshold value is, instead of the first conversion table, stored in the storage 404. According to Sepsis-3, sepsis is defined as a condition in which an infection and an acute increase in the SOFA score by 2 or more points are observed. Considering this definition, the threshold value may be set to a value, of the information (NE-WY) about the distribution width of the fluorescence intensities regarding the population of neutrophils, at which the estimated value of the difference between the SOFA score in the initial stage of hospitalization and the SOFA score in the healthy state is 2, for example. Alternatively, the threshold value may be set as appropriate.

By the main body 40, the information processing unit 4 compares the generated information (NE-WY) about the distribution width of the fluorescence intensities regarding the population of neutrophils and the threshold value read out from the storage 404 with each other. When determining that the NE-WY is equal to or larger than the threshold value, the information processing unit 4 causes, by the main body 40, the output part 42 to display, in the region 63 of the analysis result screen 6 displayed on the output part 42, flag information indicating that the estimated value of the difference between the SOFA score in the initial stage of hospitalization and the SOFA score in the healthy state is equal to or larger than the threshold value. Meanwhile, when determining that the NE-WY is smaller than the threshold value, the information processing unit 4 causes, by the main body 40, the output part 42 to display, in the region 63 of the analysis result screen 6 displayed on the output part 42, flag information indicating that the estimated value of the difference between the SOFA score in the initial stage of hospitalization and the SOFA score in the healthy state is smaller than the threshold value. Alternatively, when the NE-WY is smaller than the threshold value, the flag information may be hidden.

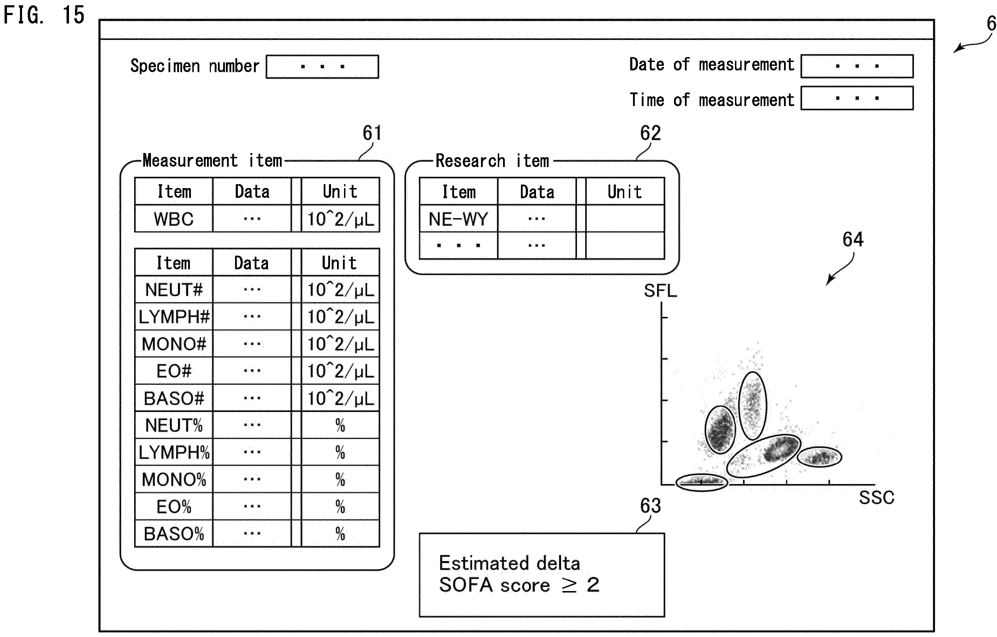

FIG. 15 shows an example of an analysis result screen regarding the blood specimen according to the fourth embodiment. FIG. 15 shows an analysis result screen 6 in a case where the NE-WY is equal to or larger than the threshold value. As shown in FIG. 15, the text "Estimated delta SOFA score≥2" is displayed in the region 63 so as to indicate that the estimated value of the difference between the SOFA score in the initial stage of hospitalization and the SOFA score in the healthy state is 2 or larger.

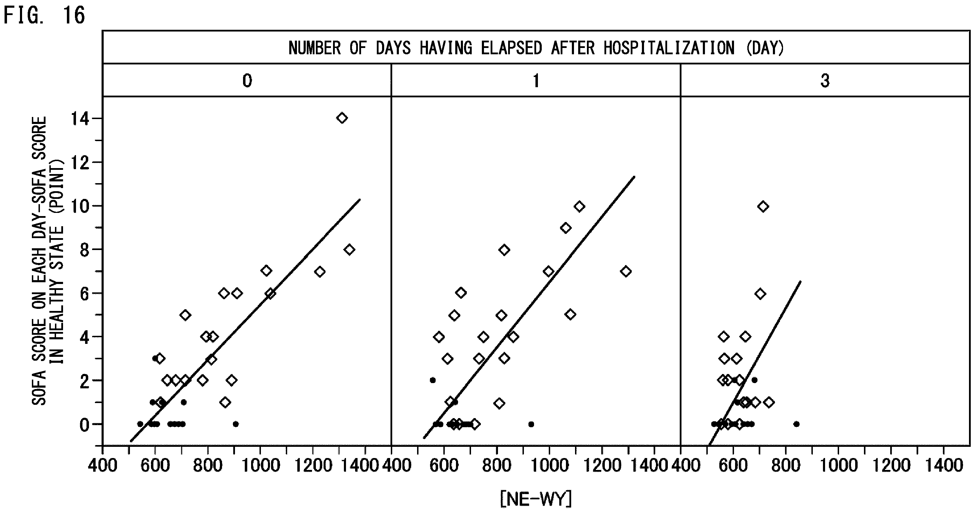

A method for setting the threshold value will be described with reference to FIG. 16.

FIG. 16 shows correlations each of which is a correlation between the information (NE-WY) about the distribution widths of the fluorescence intensities regarding the populations of neutrophils of the hospitalized patients on the corresponding day and the difference between the SOFA score on said day and the SOFA score in the healthy state. In FIG. 16, each of the leftmost graph, the center graph, and the rightmost graph is a graph indicating a correlation between the NE-WY on the corresponding one of the zeroth day after hospitalization, the first day after hospitalization, and the third day after hospitalization and the difference between the SOFA score on said day and the SOFA score in the healthy state. In each of the graphs, the white dots are data of sepsis patients, and the black dots are data of non-sepsis patients. The sepsis patients and the non-sepsis patients are the same as the respective patients in FIG. 8. The method for calculating the information (NE-WY) about the distribution width of the fluorescence intensities regarding the population of neutrophils of each of the hospitalized patients on each day, and the blood cell analyzer used for the calculation, are the same as those described above. The SOFA score in the healthy state is a SOFA score of each of the patients at the time of discharge from hospital. The difference between the SOFA score on each day and the SOFA score in the healthy state has been calculated by subtracting the SOFA score of each of the patients in the healthy state from the SOFA score of the patient on said day.

Spearman's rank correlation coefficients r each indicating the correlation between the NE-WY on the corresponding one of the initial day of (zeroth day after) hospitalization, the first day after hospitalization, and the third day after hospitalization and the difference between the SOFA score on said day and the SOFA score in the healthy state, are respectively 0.67, 0.53, and 0.23. The correlation on the initial day of (zeroth day after) hospitalization has been found to be highest, and the correlation on the first day after hospitalization has been found to be second highest. In addition, it has been found that the correlation decreases day by day after hospitalization. The reason why the correlation decreases day by day is considered to be because a therapy is performed through administration of medication so as to suppress the infection from the initial day of hospitalization, so that the infection subsides according to the therapy. In addition, the present inventor has calculated regression expressions from the data of the sepsis patients and the non-sepsis patients on the respective days shown in FIG. 16. The regression expressions for the initial day of (zeroth day after) hospitalization, the first day after hospitalization, and the third day after hospitalization, are respectively y=0.013x-7.3, y=0.015x-8.5, and y=0.022x-12.1. In each of these expressions, the NE-WY is set as an x axis, and the difference between the SOFA score on the corresponding day and the SOFA score in the healthy state is set as a y axis.

Threshold values can be obtained from the respective regression expressions in each of which the index indicating the distribution width of the fluorescence intensities regarding the population of neutrophils and the difference between a corresponding SOFA score and the SOFA score in the healthy state are associated with each other, such as ones shown in FIG. 16. For example, the NE-WY at which the difference is 2 can be set as the threshold value. This is because, according to Sepsis-3, sepsis is defined as a condition in which an infection and an acute increase in the SOFA score by 2 or more points are observed.

A threshold value can be obtained correspondingly to each of the initial day of (zeroth day after) hospitalization, the first day after hospitalization, and the third day after hospitalization and can be stored in the storage 404. The information processing unit 4 may select any of the threshold values according to the number of days having elapsed after hospitalization of the subject, and obtain flag information. For example, in a case where the present day is the initial day of hospitalization of the subject, the information processing unit 4 receives, from the host computer 5 or through the input part 41 from a user, input of information indicating that "the present day is the initial day of hospitalization of the subject". Then, the information processing unit 4 reads out, from the storage 404, the threshold value corresponding to the initial day of hospitalization, compares the threshold value and the NE-WY with each other, and causes the output part 42 to display, in the region 63 of the analysis result screen 6 displayed on the output part 42, flag information indicating "Estimated delta SOFA score≥2" or "Estimated delta SOFA score<2". Alternatively, the threshold value does not have to vary according to the number of days having elapsed after the initial day of hospitalization, and a common threshold value may be used.

In modification 1 of the present embodiment, regression expressions may be created correspondingly to respective numbers of days having elapsed after hospitalization, and computer programs including the regression expressions may be prestored in the storage 404. In this case, by the main body 40, the information processing unit 4 executes the computer program including the regression expression corresponding to the number of days having elapsed after hospitalization of the subject, and determines flag information indicating whether or not the estimated value of the difference between the SOFA score in the initial stage of hospitalization and the SOFA score in the healthy state is equal to or larger than the threshold value. The input of the number of days having elapsed after hospitalization of the subject, is received through the input part 41 or from the host computer 5.

In modification 2 of the present embodiment, a computer program including a common regression expression that does not vary according to the number of days having elapsed after hospitalization, may be stored in the storage 404. In this case, by the main body 40, the information processing unit 4 executes this computer program and determines flag information indicating whether or not the estimated value of the difference between the SOFA score in the initial stage of hospitalization and the SOFA score in the healthy state is equal to or larger than the threshold value.

As described above, the flag information in the present embodiment includes information indicating whether or not the estimated value of the difference between the SOFA score in the initial stage of hospitalization and the SOFA score in the healthy state is equal to or larger than the threshold value. Consequently, it is possible to provide a specimen analysis method and a specimen analyzer for performing assistance in a swift evaluation of a degree of severity of an organ dysfunction due to an infection, the specimen analysis method and the specimen analyzer enabling outputting of information having a high correlation with a score indicating the degree of severity of the organ dysfunction due to the infection. Specifically, it is possible to: assist a doctor in diagnosing that the subject suffers from sepsis; and assist the doctor in determining a therapeutic strategy for the subject.

### [5. Other Embodiments]

The embodiments described above are merely examples for explaining the present disclosure, and the present disclosure is not limited to these implementation examples. The present disclosure can be carried out in various aspects unless the aspects deviate from the gist of the present disclosure. For example, a combination of two or more of the first to fourth embodiments and the modifications is also encompassed in the present disclosure. Regarding the processes or operations described above, the sequences, the executing constituents, and the like of the processes or operations can be freely changed unless the change leads to a contradiction in terms of the processes or operations such as a contradiction that, in a step, data that should not be usable yet in the step is used.

### [6. Remarks]

The present disclosure includes following items 1-19.

Item 1: A specimen analysis method comprising:
measuring fluorescence signals,
   the measuring including applying light to a measurement sample containing a blood specimen and a fluorescent dye that stains white blood cells contained in the blood specimen, and detecting fluorescence from the white blood cells contained in the measurement sample to which the light has been applied,
   the measuring being performed on the basis of the detected fluorescence;
generating, on the basis of the fluorescence signals, information about a distribution width of fluorescence intensities regarding a population of neutrophils contained in the measurement sample;
determining information about a degree of severity of an organ dysfunction due to an infection on the basis of the information about the distribution width of the fluorescence intensities regarding the population of neutrophils; and
outputting the information about the degree of severity of the organ dysfunction due to the infection.

Item 2: The specimen analysis method of item 1, wherein
the information about the degree of severity of the organ dysfunction due to the infection includes flag information indicating
that a subject from whom the blood specimen has been collected is suspected of suffering from sepsis, or
a level of a possibility that the subject suffers from sepsis.

Item 3: The specimen analysis method of item 1, wherein
the information about the degree of severity of the organ dysfunction due to the infection includes an estimated score indicating the degree of severity of the organ dysfunction due to the infection.

Item 4: The specimen analysis method of item 1, wherein
the information about the degree of severity of the organ dysfunction due to the infection includes a range of an estimated score indicating the degree of severity of the organ dysfunction due to the infection.

Item 5: The specimen analysis method of item 3 or 4, wherein
the information about the distribution width of the fluorescence intensities regarding the population of neutrophils indicates the distribution width of the fluorescence intensities regarding the population of neutrophils, and,
as the distribution width becomes larger, the estimated score indicating the degree of severity of the organ dysfunction due to the infection becomes larger.

Item 6: The specimen analysis method of item 3 or 4, wherein
the estimated score indicating the degree of severity of the organ dysfunction due to the infection is an estimated value of a SOFA score.

Item 7: The specimen analysis method of item 6, wherein
the information about the degree of severity of the organ dysfunction due to the infection includes information indicating whether or not an estimated value of a difference between a SOFA score in an initial stage of hospitalization and a SOFA score in a healthy state is equal to or larger than a threshold value.

Item 8: The specimen analysis method of item 1, wherein
the measuring further includes preparing the measurement sample.

Item 9: The specimen analysis method of item 1, wherein
the measuring further includes causing the measurement sample to flow through a flow cell, and
the applying included in the measuring includes applying the light to the measurement sample flowing through the flow cell.

Item 10: A specimen analyzer comprising:
a measurement part configured to
   apply light to a measurement sample containing a blood specimen and a fluorescent dye that stains white blood cells contained in the blood specimen,
   detect fluorescence from the white blood cells contained in the measurement sample to which the light has been applied, and
   measure fluorescence signals on the basis of the detected fluorescence;
an information processing part configured to
   generate, on the basis of the fluorescence signals, information about a distribution width of fluorescence intensities regarding a population of neutrophils contained in the measurement sample and
   determine information about a degree of severity of an organ dysfunction due to an infection on the basis of the information about the distribution width of the fluorescence intensities regarding the population of neutrophils; and
an output part configured to output the information about the degree of severity of the organ dysfunction due to the infection.

Item 11: The specimen analyzer of item 10, wherein
the information about the degree of severity of the organ dysfunction due to the infection includes flag information indicating
that a subject from whom the blood specimen has been collected is suspected of suffering from sepsis, or
a level of a possibility that the subject suffers from sepsis.

Item 12: The specimen analyzer of item 10, wherein
the information about the degree of severity of the organ dysfunction due to the infection includes an estimated score indicating the degree of severity of the organ dysfunction due to the infection.

Item 13: The specimen analyzer of item 10, wherein
the information about the degree of severity of the organ dysfunction due to the infection includes a range of an estimated score indicating the degree of severity of the organ dysfunction due to the infection.

Item 14: The specimen analyzer of item 12 or 13, wherein
the information about the distribution width of the fluorescence intensities regarding the population of neutrophils indicates the distribution width of the fluorescence intensities regarding the population of neutrophils, and,
as the distribution width becomes larger, the estimated score indicating the degree of severity of the organ dysfunction due to the infection becomes larger.

Item 15: The specimen analyzer of item 14, wherein
the estimated score indicating the degree of severity of the organ dysfunction due to the infection is an estimated value of a SOFA score.

Item 16: The specimen analyzer of item 12 or 13, wherein
the information about the degree of severity of the organ dysfunction due to the infection includes information indicating whether or not an estimated value of a difference between a SOFA score in an initial stage of hospitalization and a SOFA score in a healthy state is equal to or larger than a threshold value.

Item 17: The specimen analyzer of item 10, wherein
the measurement part includes a preparation part configured to prepare the measurement sample.

Item 18: The specimen analyzer of item 10, wherein
the measurement part includes a flow cell configured to allow the measurement sample to flow therethrough and applies the light to the measurement sample flowing through the flow cell.

Item 19: The specimen analyzer of item 10, comprising at least one of the following configurations [11] to [18]:
[11] a configuration in which the information about the degree of severity of the organ dysfunction due to the infection includes flag information indicating that a subject from whom the blood specimen has been collected is suspected of suffering from sepsis;
[12] a configuration in which the information about the degree of severity of the organ dysfunction due to the infection includes an estimated score indicating the degree of severity of the organ dysfunction due to the infection;
[13] a configuration in which the information about the degree of severity of the organ dysfunction due to the infection includes a range of an estimated score indicating the degree of severity of the organ dysfunction due to the infection;
[14] a configuration in which the information about the distribution width of the fluorescence intensities regarding the population of neutrophils indicates the distribution width of the fluorescence intensities regarding the population of neutrophils, and, as the distribution width becomes larger, the estimated score indicating the degree of severity of the organ dysfunction due to the infection becomes larger;
[15] a configuration in which the estimated score indicating the degree of severity of the organ dysfunction due to the infection is an estimated value of a SOFA score;
[16] a configuration in which the flag information includes information indicating whether or not an estimated value of a difference between a SOFA score in an initial stage of hospitalization and a SOFA score in a healthy state is equal to or larger than a threshold value;
[17] a configuration in which the measurement part includes a preparation part configured to prepare the measurement sample; and
[18] a configuration in which the measurement part includes a flow cell configured to allow the measurement sample to flow therethrough and applies the light to the measurement sample flowing through the flow cell.

### DESCRIPTION OF THE REFERENCE CHARACTERS

1 blood cell analyzer
2 measurement unit
21 hand part
22 specimen container setting part
23 barcode unit
24 specimen suction part
25 sample preparation part
26 detection part
D optical detector
D1 flow cell
D2 sheath flow system
D3 beam spot forming system
D31 semiconductor laser
D32 collimator lens
D33 condenser lens
D34 beam stopper
D4 forward scattered light receiving system
D41 forward condenser lens
D42 pinhole
D43 photodiode
D5 side scattered light receiving system
D51 side condenser lens
D52 dichroic mirror
D53 photodiode
D6 fluorescence receiving system
D61 spectral filter
D62 photodiode
D7 signal processing circuit
3 transport unit
4 information processing unit
40 main body
401 CPU
402 ROM
403 RAM
404 storage
404a program
405 read-out device
405a storage medium
406 image output interface
407 input/output interface
408 communication interface
41 input part
42 output part
5 host computer

## Claims

1. A specimen analyzer comprising:
a measurement part configured to
apply light to a measurement sample containing a blood specimen and a fluorescent dye that stains white blood cells contained in the blood specimen,
detect fluorescence from the white blood cells contained in the measurement sample to which the light has been applied, and
measure fluorescence signals on the basis of the detected fluorescence;
an information processing part configured to
generate, on the basis of the fluorescence signals, information about a distribution width of fluorescence intensities regarding a population of neutrophils contained in the measurement sample, and
determine information about a degree of severity of an organ dysfunction due to an infection on the basis of the information about the distribution width of the fluorescence intensities regarding the population of neutrophils; and
an output part configured to output the information about the degree of severity of the organ dysfunction due to the infection.

2. The specimen analyzer of claim 1, wherein
the information about the degree of severity of the organ dysfunction due to the infection includes flag information indicates
that a subject from whom the blood specimen has been collected is suspected of suffering from sepsis, or
a level of a possibility that the subject suffers from sepsis.

3. The specimen analyzer of claim 1, wherein
the information about the degree of severity of the organ dysfunction due to the infection includes an estimated score indicating the degree of severity of the organ dysfunction due to the infection.

4. The specimen analyzer of claim 1, wherein
the information about the degree of severity of the organ dysfunction due to the infection includes a range of an estimated score indicating the degree of severity of the organ dysfunction due to the infection.

5. The specimen analyzer of claim 3 or 4, wherein
the information about the distribution width of the fluorescence intensities regarding the population of neutrophils indicates the distribution width of the fluorescence intensities regarding the population of neutrophils, and,
as the distribution width becomes larger, the estimated score indicating the degree of severity of the organ dysfunction due to the infection becomes larger.

6. The specimen analyzer of claim 5, wherein
the estimated score indicating the degree of severity of the organ dysfunction due to the infection is an estimated value of a SOFA score.

7. The specimen analyzer of claim 6, wherein
the information about the degree of severity of the organ dysfunction due to the infection includes information indicating whether or not an estimated value of a difference between the SOFA score in an initial stage of hospitalization and the SOFA score in a healthy state is equal to or larger than a threshold value.

8. The specimen analyzer of claim 1, wherein
the measurement part includes a preparation part configured to prepare the measurement sample.

9. The specimen analyzer of claim 1, wherein
the measurement part includes a flow cell configured to allow the measurement sample to flow therethrough and applies the light to the measurement sample flowing through the flow cell.

10. A specimen analysis method comprising:
applying light to a measurement sample containing a blood specimen and a fluorescent dye that stains white blood cells contained in the blood specimen, detecting fluorescence from the white blood cells contained in the measurement sample to which the light has been applied, and measuring fluorescence signals on the basis of the detected fluorescence;
generating, on the basis of the fluorescence signals, information about a distribution width of fluorescence intensities regarding a population of neutrophils contained in the measurement sample;
determining information about a degree of severity of an organ dysfunction due to an infection on the basis of the information about the distribution width of the fluorescence intensities regarding the population of neutrophils; and
outputting the information about the degree of severity of the organ dysfunction due to the infection.

11. The specimen analysis method of claim 10, wherein
the information about the degree of severity of the organ dysfunction due to the infection includes flag information indicates
that a subject from whom the blood specimen has been collected is suspected of suffering from sepsis, or
a level of a possibility that the subject suffers from sepsis.

12. The specimen analysis method of claim 10, wherein
the information about the degree of severity of the organ dysfunction due to the infection includes
an estimated score indicating the degree of severity of the organ dysfunction due to the infection, or
a range of an estimated score indicating the degree of severity of the organ dysfunction due to the infection.

13. The specimen analysis method of claim 12, wherein
the information about the distribution width of the fluorescence intensities regarding the population of neutrophils indicates the distribution width of the fluorescence intensities regarding the population of neutrophils, and,
as the distribution width becomes larger, the estimated score indicating the degree of severity of the organ dysfunction due to the infection becomes larger.

14. The specimen analysis method of claim 12 or 13, wherein
the estimated score indicating the degree of severity of the organ dysfunction due to the infection is an estimated value of a SOFA score.

15. The specimen analysis method of claim 14, wherein
the information about the degree of severity of the organ dysfunction due to the infection includes information indicating whether or not an estimated value of a difference between the SOFA score in an initial stage of hospitalization and the SOFA score in a healthy state is equal to or larger than a threshold value.
